# EUROPEAN PATENT APPLICATION

(11) **EP 4 631 545 A1**
(43) Date of publication of application: **15.10.2025**
(21) Application number: 23900667.9
(22) Date of filing: 05.12.2023
(51) Int. Cl.: A61M 1/36, A61K 38/17, A61P 1/04, A61P 1/16, A61P 29/00, C07K 1/22, C07K 17/00

(54) **METHOD FOR TREATING ULCERATIVE COLITIS OR PRIMARY SCLEROSING CHOLANGITIS**

(30) Priority: 05.12.2022 JP 2022194318
(71) Applicant: Kyoto University, Kyoto-shi, Kyoto 606-8501 (JP); Link Therapeutics Inc., Kyoto-shi, Kyoto 606-8304 (JP)
(72) Inventor: SHIOKAWA, Masahiro, Kyoto-shi, Kyoto 606-8501 (JP); KUWADA, Takeshi, Kyoto-shi, Kyoto 606-8501 (JP); OTA, Sakiko, Kyoto-shi, Kyoto 606-8501 (JP); YOSHIDA, Hiroyuki, Kyoto-shi, Kyoto 606-8501 (JP); NISHIKAWA, Yoshihiro, Kyoto-shi, Kyoto 606-8501 (JP); MURAMOTO, Yuya, Kyoto-shi, Kyoto 606-8501 (JP); TAKIMOTO, Ikuhisa, Kyoto-shi, Kyoto 606-8501 (JP); SENO, Hiroshi, Kyoto-shi, Kyoto 606-8501 (JP); CHIBA, Tsutomu, Kyoto-shi, Kyoto 606-8501 (JP); KINOSHITA, Koshi, Kyoto-shi, Kyoto 606-8304 (JP); AOSASA, Masayoshi, Kyoto-shi, Kyoto 606-8304 (JP); KURIHARA, Daichi, Kyoto-shi, Kyoto 606-8304 (JP); ABE, Yoshiko, Kyoto-shi, Kyoto 606-8304 (JP)
(74) Representative: Hoffmann Eitle
(86) International application number: PCT/JP2023/043549
(87) International publication number: WO 2024/122553

(57) **Abstract**

Provided are, inter alia, a system for treating ulcerative colitis or primary sclerosing cholangitis including a means for removing anti-integrin αvβ6 antibodies or anti-integrin αvβ6 antibody-producing B-cells that produce the anti-integrin αvβ6 antibodies, the anti-integrin αvβ6 antibodies having an activity of competing with fibronectin for binding to integrin αvβ6 and being specifically produced in patients having ulcerative colitis or primary sclerosing cholangitis.

## Description

### TECHNICAL FIELD

The present invention relates to a method, a system, etc. for treating ulcerative colitis (hereinafter also referred to as "UC") or primary sclerosing cholangitis (hereinafter also referred to as "PSC").

### BACKGROUND ART

Ulcerative colitis is a refractory and unexplained disease accompanied by symptoms of diarrhea, bloody stool, abdominal pain, etc., and continuous erosions or inflammatory ulcerations extending from the rectum are found in the mucous membrane of large intestine. Ulcerative colitis develops in people of all ages from mainly young people to the elderly. Ulcerative colitis requires long-term treatment due to repeated remission and relapse. The number of patients with ulcerative colitis is very large and has been increasing worldwide in recent years.

Primary sclerosing cholangitis is a progressive chronic liver disease that causes multiple and diffuse fibrous stenosis in bile ducts inside and outside the liver. Approximately half of patients with primary sclerosing cholangitis progress to cirrhosis within 10 years. In cases where primary sclerosing cholangitis progresses to cirrhosis, though liver transplantation is required, many patients cannot receive a liver transplant and then die. Although primary sclerosing cholangitis is believed to be a multifactorial disease involving immunological abnormalities, the cause remains unknown. Primary sclerosing cholangitis often coexists with inflammatory bowel disease (IBD). Among inflammatory bowel diseases, ulcerative colitis is present in a certain percentage of patients with primary sclerosing cholangitis (in Japan, it has been reported that approximately 40% of patients with primary sclerosing cholangitis also have ulcerative colitis, and that in particular, approximately 60% of young patients with primary sclerosing cholangitis also have ulcerative colitis. In Europe and the United States, it has been reported that approximately 70-80% of patients with primary sclerosing cholangitis also have ulcerative colitis.

Medical treatment of ulcerative colitis depends on the severity of symptoms. Specifically, 5-aminosalicylic acid preparations are used in mild cases, immunosuppressants such as steroids are used in moderate cases, and cytapheresis, biological preparations such as anti-TNFα antibodies, JAK inhibitors, etc. are used in moderate to severe cases (for example, regarding anti-TNFα antibodies, see Non-Patent Document 1, website name: UpToDate, [searched on November 28, 2022], Internet <URL: https://www.uptodate.com/contents/search?search=%E6%BD%BO%E 7%98%8D%E6%80%A7%E5%A4%A7%E8%85%B8%E7%82%8E%E3%80%80TNF&sp= 0&searchType=PLAIN_TEXT&source=USER_I NPUT&searchControl=TOP_PULLDOWN&searchOffset=1&autoComplete =false&language=en&max=10&index=&autoCompleteTerm=>). However, ulcerative colitis cannot yet be completely cured by medical treatment. In addition, various therapeutic agents for the medical treatment are associated with various side effects. For example, regarding anti-TNFα antibodies used to treat moderate to severe ulcerative colitis, when the function of TNFα is suppressed with an anti-TNFα antibody in the treatment of ulcerative colitis, side effects such as infections and malignant tumors occur. Indeed, anti-TNFα antibodies are shown to have a therapeutic effect on ulcerative colitis. However, since the target TNF-α itself plays a central role in the immune system including infection defense and anti-tumor action to support a wide range of biological functions, the wide range of side effects as described above occur when the function of TNF-α which is required under normal conditions is impaired.

Medical treatment for primary sclerosing cholangitis involves administration of drugs such as ursodeoxycholic acid. However, there are currently no sufficient data to determine that the drug administration improves long-term prognosis. For this reason, novel treatments are desperately needed.

### PRIOR ART DOCUMENT

### NON-PATENT DOCUMENT

Non-Patent Document 1: Nature Reviews Immunology volume 15, pages 362-374, (2015)

### SUMMARY OF THE INVENTION

### PROBLEMS TO BE SOLVED BY THE INVENTION

Therefore, an object of the present invention is to develop a novel method for treating ulcerative colitis or primary sclerosing cholangitis with reduced side effects as compared to conventional methods.

### SOLUTIONS TO THE PROBLEMS

In order to solve the above-mentioned problems, as a result of intensive research, the inventors surprisingly found that anti-integrin αvβ6 autoantibodies which are specifically produced in patients with ulcerative colitis or primary sclerosing cholangitis are involved in the pathogenesis of these diseases, and that these diseases can be treated by targeting the autoantibodies. Thus the present invention was completed.

The present invention provides the following aspects.
(1) A system for treating ulcerative colitis or primary sclerosing cholangitis, the system comprising a means for removing an anti-integrin αvβ6 antibody or an anti-integrin αvβ6 antibody-producing B cell that produces the anti-integrin αvβ6 antibody, wherein the anti-integrin αvβ6 antibody has activity competing with fibronectin for binding to integrin αvβ6 and is specifically produced in a subject suffering from ulcerative colitis or primary sclerosing cholangitis.
(2) The system according to (1), wherein the means comprises a substance that specifically binds to the anti-integrin αvβ6 antibody or the anti-integrin αvβ6 antibody-producing B cell.
(3) The system according to (2), wherein the substance is a fragment or an entirety of integrin αvβ6 protein.
(4) The system of (3), which comprises a column carrying a fragment or an entirety of integrin αvβ6 protein.
(5) A solid carrier for the treatment of ulcerative colitis or primary sclerosing cholangitis, the solid carrier comprising a fragment or an entirety of integrin αvβ6 protein capable of specifically binding to an anti-integrin αvβ6 antibody or an anti-integrin αvβ6 antibody-producing B cell that produces the anti-integrin αvβ6 antibody, and thereby being capable of adsorbing the anti-integrin αvβ6 antibody or the anti-integrin αvβ6 antibody-producing B cell, wherein the anti-integrin αvβ6 antibody has activity competing with fibronectin for binding to integrin αvβ6 and is specifically produced in a subject suffering from ulcerative colitis or primary sclerosing cholangitis.
(6) The solid carrier according to (5), wherein the anti-integrin αvβ6 antibody comprises a heavy chain CDR2 or CDR3 comprising any one amino acid sequence selected from RGD, RGRD, or RGSGD, and the fragment or entirety of integrin αvβ6 protein has a binding dissociation constant (KD value) of 100 nM or less for the anti-integrin αvβ6 antibody.
(7) The solid carrier according to (5) or (6), wherein the integrin αvβ6 protein is immobilized at a weight equal to or greater than twice the weight of the antibody to be captured, or the fragment of integrin αvβ6 protein is immobilized at a weight equal to or greater than a value obtained by multiplying twice the weight of the antibody to be captured by a proportion of the molecular weight of the fragment to the molecular weight of the entirety of integrin αvβ6.
(8) A column comprising the solid carrier according to any one of (5) to (7).
(9) The column according to (8), which comprises the solid carrier such that the weight of the integrin αvβ6 protein per column is at least twice the weight of the antibody to be captured, or the weight of the fragment of integrin αvβ6 protein per column is at least a value obtained by multiplying twice the weight of the antibody to be captured by a proportion of the molecular weight of the fragment to the molecular weight of the entirety of integrin αvβ6.
(10) The column according to (8) or (9), which is used for apheresis.
(11) A method for producing an animal model of primary sclerosing cholangitis, the method comprising immunizing a non-human animal with a fragment or an entirety of integrin αvβ6 protein.
(12) A method for producing an animal model of primary sclerosing cholangitis, the method comprising knocking out an integrin β6 gene in a non-human animal.
(13) A method for producing an animal model of an ulcerative colitis or primary sclerosing cholangitis, the method comprising administering to a non-human animal an anti-integrin αvβ6 antibody derived from a patient with ulcerative colitis or primary sclerosing cholangitis.
(14) The method according to (13), wherein the antibody is a serum or a monoclonal antibody.
(15) An animal model of ulcerative colitis having a symptom of ulcerative colitis.
(16) An animal model of primary sclerosing cholangitis having a symptom of primary sclerosing cholangitis.
(17) The animal model of primary sclerosing cholangitis according to (16), which is a knockout mouse lacking the function of integrin αvβ6.
(18) A method of screening for a therapeutic or preventive drug for ulcerative colitis or primary sclerosing cholangitis, the method comprising administering a candidate substance to the animal model according to any one of (15) to (17).
(19) A method for treating ulcerative colitis or primary sclerosing cholangitis, the method comprising removing an anti-integrin αvβ6 antibody or an anti-integrin αvβ6 antibody-producing B cell that produces the anti-integrin αvβ6 antibody, wherein the anti-integrin αvβ6 antibody has activity competing with fibronectin for binding to integrin αvβ6 and is specifically produced in a subject suffering from ulcerative colitis or primary sclerosing cholangitis.
(20) A method for removing an anti-integrin αvβ6 antibody or an anti-integrin αvβ6 antibody-producing B cell that produces the anti-integrin αvβ6 antibody from a subject, the method comprising contacting a sample derived from the subject with a substance that specifically binds to an anti-integrin αvβ6 antibody or an anti-integrin αvβ6 antibody-producing B cell that produces the anti-integrin αvβ6 antibody, wherein the anti-integrin αvβ6 antibody has activity competing with fibronectin for binding to integrin αvβ6 and is specifically produced in a subject suffering from ulcerative colitis or primary sclerosing cholangitis.

### EFFECTS OF THE INVENTION

According to the present invention, it has surprisingly been found that the presence of anti-integrin αvβ6 autoantibodies is involved in the pathogenesis of ulcerative colitis and primary sclerosing cholangitis. Therefore, according to the present invention, it is possible to treat patients with ulcerative colitis or primary sclerosing cholangitis by removing anti-integrin αvβ6 autoantibodies and B cells that produce said antibodies in patients with ulcerative colitis or primary sclerosing cholangitis. The present invention also provides a treatment system for patients with ulcerative colitis or primary sclerosing cholangitis that can be utilized for the treatment.

The present invention is based on a concept that is completely different from the conventional standard method. Thus, the present invention provides a novel therapeutic means for patients for whom the conventional treatment methods have not been effective. Existing therapeutic drugs for ulcerative colitis (e.g., anti-TNFα antibodies) have significant side effects because they target molecules that are also present in healthy individuals. However, anti-integrin αvβ6 autoantibodies targeted by the present invention are not present or are barely present in healthy individuals. Therefore, elimination of anti-integrin αvβ6 autoantibodies does not affect health, and has probably minimal side effects.

### BRIEF DESCRIPTION OF THE DRAWINGS

[Fig. 1] Figure 1 shows results of inhibition assay of integrin αvβ6 binding to fibronectin by autoantibodies derived from patients with primary sclerosing cholangitis. The vertical axis indicates binding inhibition rates (%).
[Fig.2] Figure 2 shows dose-dependent inhibition of binding of integrin αvβ6 to fibronectin by autoantibodies derived from patients with primary sclerosing cholangitis. The vertical axis indicates binding inhibition rates (%). The horizontal axis indicates dilution rates of patient- or control-derived IgG used in the assay.
[Fig. 3] Figure 3 shows a correlation between the titers of autoantibodies derived from patients with primary sclerosing cholangitis against integrin αvβ6 and the blocking activity of integrin αvβ6-fibronectin binding.
[Fig. 4] Figure 4 shows results of inhibition assay of binding of autoantibodies derived from patients with primary sclerosing cholangitis to integrin αvβ6 by addition of peptide RGDS. The vertical axis indicates the titers (A⁴⁵⁰) of autoantibodies detected on a solid phase. The horizontal axis indicates the addition amounts of the peptide (µg/mL).
[Fig. 5] Figure 5 shows results of inhibition assay of binding of autoantibodies derived from patients with primary sclerosing cholangitis to integrin αvβ6 by addition of peptide RGES. The vertical axis indicates the titers (A⁴⁵⁰) of autoantibodies detected on a solid phase. The horizontal axis indicates the addition amounts of the peptide (µg/mL).
[Fig. 6] Figure 6 shows the inhibitory effect of monoclonal anti-integrin αvβ6 antibodies derived from patients with ulcerative colitis or primary sclerosing cholangitis on the integrin αvβ6-fibronectin binding. In the figure, "UC antibody 1," "UC antibody 2," "UC antibody 3," "UC antibody 4," "UC antibody 5," "UC antibody 6," "UC antibody 7," and "UC antibody 8" represent eight monoclonal anti-integrin αvβ6 antibodies established from the blood of UC patients. "PSC antibody 1" refers to a monoclonal anti-integrin αvβ6 antibody established from the blood of a PSC patient.
[Fig. 7] Figure 7 shows photomicrographs of bile ducts in integrin αvβ6-immunized mice and control-immunized mice.
[Fig. 8] Figure 8 shows photomicrographs of bile ducts in integrin αvβ6-immunized mice to which various depleting antibodies were administered.
[Fig. 9] Figure 9 is a photomicrograph of a bile duct in a mouse lacking the function of integrin αvβ6.
[Fig. 10] Figure 10 shows concentration-dependent adsorption rates of a column carrying integrin αvβ6.
[Fig. 11] Figure 11 shows adsorption rates of antibodies in the sera of patients with ulcerative colitis on a column carrying integrin αvβ6.
[Fig. 12] Figure 12 shows adsorption rates of antibodies in the sera of patients with primary sclerosing cholangitis on a column carrying integrin αvβ6.
[Fig. 13] Figure 13 shows results of tests for ligand protein-specificity of monoclonal anti-integrin αvβ6 autoantibodies derived from ulcerative colitis patients.
[Fig. 14] Figure 14 shows adsorption rates of a monoclonal autoantibody derived from an ulcerative colitis patient and autoantibodies derived from the serum of an ulcerative colitis patient on a carrier comprising integrin αvβ6 immobilized by NHS covalent bonding.
[Fig. 15] Figure 15 shows adsorption rates of a monoclonal autoantibody derived from an ulcerative colitis patient on a carrier comprising integrin αvβ6 immobilized by avidin-biotin binding.
[Fig. 16] Figure 16 shows a comparison of the adsorption amounts of a monoclonal anti-integrin αvβ6 autoantibody derived from an ulcerative colitis patient to integrin αvβ6 immobilized by avidin-biotin binding and integrin αvβ6 immobilized by covalent bonding.

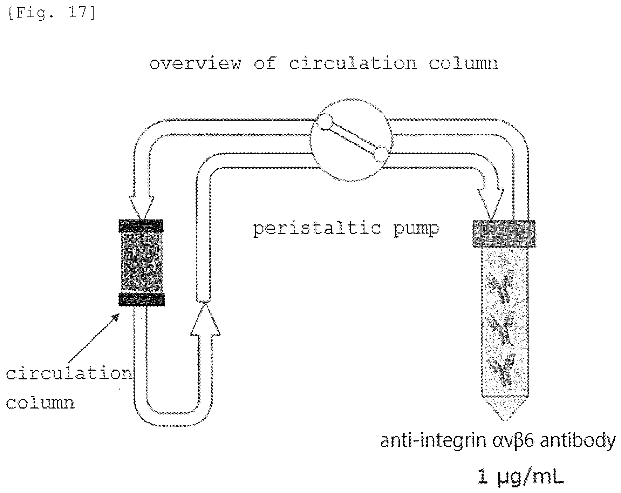

[Fig. 17] Figure 17 shows an overview of an example of a circulation column system.
[Fig. 18-1] Figure 18-1 shows adsorption rates of anti-integrin αvβ6 antibodies in a circulation column packed with an integrin αvβ6-immobilized adsorbent.
[Fig. 18-2] Figure 18-2 shows adsorption rates of anti-integrin αvβ6 antibodies in a circulation column packed with a BSA-immobilized adsorbent.
[Fig. 19] Figure 19 shows photomicrographs of the large intestines of mice administered with a UC patient serum and a PSC patient serum.
[Fig. 20] Figure 20 shows photomicrographs of the bile ducts of mice administered with a UC patient serum and a PSC patient serum.
[Fig. 21] Figure 21 shows photomicrographs of the large intestine of DSS-administered mice administered with a UC patient serum and a PSC patient serum.
[Fig. 22] Figure 22 shows photomicrographs of the bile ducts of DSS-administered mice administered with a UC patient serum and a PSC patient serum.
[Fig. 23] Figure 23 shows a photomicrograph of the large intestine of a mouse administered with an anti-integrin αvβ6 monoclonal antibody derived from a UC patient.
[Fig. 24] Figure 24 shows photomicrographs of the large intestine of a DSS-administered mouse administered with an anti-integrin αvβ6 monoclonal antibody derived from a UC patient.

### MODE FOR CARRYING OUT INVENTION

The inventors previously showed that anti-integrin αvβ6 antibodies were specifically produced in patients with ulcerative colitis or primary sclerosing cholangitis (WO2020/141608). Integrin αvβ6 is known to bind to ligands such as fibronectin by recognizing tripeptide motif RGD. Furthermore, the previous study by the inventors showed that, in an in vitro experiment using an ELISA method, the fibronectin-integrin αvβ6 binding was inhibited in a concentration-dependent manner by addition of autoantibodies derived from ulcerative colitis patients, that the inhibitory activity correlated with the titers of anti-integrin αvβ6 antibodies derived from the patients, and that the binding of the patient-derived autoantibodies to integrin αvβ6 was inhibited in a concentration-dependent manner by the RGD peptide (Gastroenterology Vol. 160, No. 7, June 2021, Pages 2383-2394).

Thus, at this time, the inventors hypothesized that ulcerative colitis or primary sclerosing cholangitis is caused by inhibition of the binding between integrin αvβ6 expressed in the colon or bile duct epithelial cell layer and fibronectin in the connective tissue layer via the RGD tripeptide motif by anti-integrin αvβ6 autoantibodies, and then, investigated the pathogenesis of ulcerative colitis and primary sclerosing cholangitis. As a result, the present invention actually demonstrated that anti-integrin αvβ6 autoantibodies derived from patients with ulcerative colitis and primary sclerosing cholangitis contain the RGD tripeptide motif or a sequence similar thereto in the complementarity determining regions (CDR) that forms the antigen-binding site, i.e., the epitope of the antibodies is located in the RGD-binding site of integrin αvβ6 (Example 2). Furthermore, it was demonstrated that autoantibodies derived from patients with primary sclerosing cholangitis inhibit the binding of integrin αvβ6 to fibronectin in a concentration-dependent manner, that the inhibitory activity correlates with the titers of anti-integrin αvβ6 antibodies derived from the patients, and that the binding of the patient-derived autoantibodies to integrin αvβ6 is inhibited by the RGD peptide in a concentration-dependent manner (Example 1). Furthermore, it was demonstrated that primary sclerosing cholangitis develops by immunizing mice with integrin αvβ6 to induce production of anti-integrin αvβ6 antibodies, and that the pathological condition is alleviated by removal of the antibodies (Example 3). Furthermore, the present inventors generated integrin αvβ6 gene knockout mice and demonstrated that the mice exhibited findings consistent with primary sclerosing cholangitis (Example 4). Thus, according to the present invention, it was found that the anti-integrin αvβ6 antibody is involved in the pathogenesis of ulcerative colitis and primary sclerosing cholangitis. Based on this finding, the present invention provides a technology comprising removing an anti-integrin αvβ6 antibody or an anti-integrin αvβ6 antibody-producing B cell that produces the anti-integrin αvβ6 antibody, wherein the anti-integrin αvβ6 antibody has activity competing with fibronectin for binding to integrin αvβ6 and is specifically produced in a subject suffering from ulcerative colitis or primary sclerosing cholangitis.

### <1. Subjects>

The subjects to which the present invention is applicable are animals suffering from ulcerative colitis or primary sclerosing cholangitis and in need of treatment. The subject is preferably an animal positive for an anti-integrin αvβ6 antibody in blood (an animal that produces an anti-integrin αvβ6 antibody in the body). More preferably, the subject is an animal that is positive for an anti-integrin αvβ6 antibody in blood and does not improve with other therapies (e.g., conventional therapies). The type of animal is not particularly limited and may be a human or a non-human mammal. Preferably the animal is a human.

### <2. Anti-integrin αvβ6 antibody>

Integrin in the natural form is heterodimeric protein consisting of two subunit chains, an α chain and a β chain. Known α chains are α1 to α11, αv, αX, αM, αL, αD, αE, and αIIb, and known β chains are β1 to β8. There are multiple integrin isoforms with different combinations of α chains and β chains. Integrin is present on the surfaces of epithelial cells, and binds to extracellular matrix proteins such as laminin and fibronectin on the surfaces of connective tissues, thereby playing an important role in cell adhesion.

Integrin αvβ6 consists of a heterodimeric molecule containing αv as the α chain and β6 as the β chain. Integrin αvβ6 is hardly expressed in normal tissues, and is expressed on the surfaces of epithelial cells upon inflammatory stimulation.

As used herein, the term "anti-integrin αvβ6 antibody" refers to an antibody that specifically binds to integrin αvβ6 or a fragment thereof.

Therefore, the anti-integrin αvβ6 antibodies to be removed in the present invention are anti-integrin αvβ6 antibodies that are specifically produced in subjects suffering from ulcerative colitis or primary sclerosing cholangitis. The anti-integrin αvβ6 antibodies are autoantibodies. The anti-integrin αvβ6 autoantibodies are not present or barely present in healthy individuals.

Furthermore, the anti-integrin αvβ6 antibody has an activity of competing with fibronectin for binding to integrin αvβ6. That is, the anti-integrin αvβ6 antibody is an antibody that specifically binds to integrin αvβ6 and inhibits the binding of the integrin αvβ6 to fibronectin. As used herein, "inhibition" encompasses suppression, reduction, and loss.

The anti-integrin αvβ6 antibody preferably inhibits or suppresses the binding between integrin αvβ6 and fibronectin via the RGD tripeptide motif of fibronectin. More preferably, the anti-integrin αvβ6 antibody is an antibody that binds to an epitope containing the RGD-binding domain on integrin αvβ6.

Even more preferably, the anti-integrin αvβ6 antibody comprises the RGD peptide sequence or a similar sequence thereto. Examples of the similar sequence to the RGD peptide include, but not limited to, RGRD (SEQ ID NO: 7), RGSGD (SEQ ID NO: 8), RED, KGD, and SGD. The RGD peptide sequence or a similar sequence thereto is contained preferably in the heavy and/or light chain complementarity determining region (CDR), more preferably in the heavy and/or light chain CDR2 or CDR3, particularly preferably in the heavy chain CDR2 or CDR3, and even more preferably in the heavy chain CDR3 of the anti-integrin αvβ6 antibody. For example, the anti-integrin αvβ6 antibody comprises a heavy chain CDR3 comprising the sequence set forth as AKVIPRIRGSGDKAGIKDYYYYGMDV (SEQ ID NO: 3), ATDRPLKLRGRDYNYYVMDV (SEQ ID NO: 4), AKDRGRRGDSGWYRHFDY (SEQ ID NO: 5), or ARDRGFRGDTAMIKGGMDV (SEQ ID NO: 6).

The binding dissociation constant (KD value) of the anti-integrin αvβ6 antibody to integrin αvβ6 or a fragment thereof is preferably 100 nM or less, more preferably 50 nM or less, ord particularly preferably 25 nM or less. Preferably, an anti-integrin αvβ6 antibody comprising the RGD peptide sequence or a similar sequence thereto, or more preferably an anti-integrin αvβ6 antibody comprising the RGD peptide sequence or a similar sequence thereto in the heavy or light chain CDR2 or CDR3, particularly preferably in the heavy chain CDR2 or CDR3, or even more preferably in the heavy chain CDR3 binds to integrin αvβ6 or a fragment thereof with a KD value of, for example, 100 nM or less, preferably 50 nM or less, and more preferably 25 nM or less. Furthermore, the anti-integrin αvβ6 antibody preferably exhibits the property of being captured with an adsorption rate of 50% or more by integrin αvβ6 in an amount equal to or greater than twice the weight of the antibody to be captured, or by the fragment of integrin αvβ6 protein in an amount equal to or greater than a value obtained by multiplying twice the weight of the antibody to be captured by a proportion of the molecular weight of the fragment to the molecular weight of the entirety of integrin αvβ6. As used herein, the term "adsorption rate" refers to a proportion of the amount of antigen-bound antibody in the amount of antibody added in an antigen-antibody reaction system. For example, an antibody is added to a reaction system containing an antigen, and then the amount of free antibody (the amount of antibody not bound to the antigen) is measured, and the proportion of the amount of antibody bound to the antigen in the amount of antibody added to the reaction system is calculated. The amounts of integrin αvβ6 antibodies in UC and PSC patient sera were found to be approximately 5-10 µg/mL. When used in clinical practice for patients with high antibody titers, a large amount of adsorbent may be used.

### <3. Anti-integrin αvβ6 antibody-producing B cell>

As used herein, the term "anti-integrin αvβ6 antibody-producing B cell" refers to B cells that produce anti-integrin αvβ6 antibodies. B cells that produce antibodies express B cell antigen receptors (membrane-type immunoglobulin) having the same structure as the antibodies on the cell surfaces. Therefore, the anti-integrin αvβ6 antibody-producing B cells express the membrane-type anti-integrin αvβ6 antibodies. Therefore, any substance that specifically binds to an anti-integrin αvβ6 antibody specifically binds to the anti-integrin αvβ6 antibody-producing B cells.

### <4. Method for treating ulcerative colitis or primary sclerosing cholangitis>

The treatment method of the present invention comprises removing an anti-integrin αvβ6 antibody or an anti-integrin αvβ6 antibody-producing B cell that produces the anti-integrin αvβ6 antibody, wherein the anti-integrin αvβ6 antibody has activity competing with fibronectin for binding to integrin αvβ6 and is specifically produced in a subject suffering from ulcerative colitis or primary sclerosing cholangitis.

As used herein, the "removal" of the anti-integrin αvβ6 antibody or the anti-integrin αvβ6 antibody-producing B cell means that the antibody or B cell is removed from the body of the subject, and for example, if the antibody or B cell is present in the subject's blood, some or all of the antibodies or B cells present in the blood are removed from the blood.

The removal of the anti-integrin αvβ6 antibody or the anti-integrin αvβ6 antibody-producing B cell may be carried out by any method. In order to remove the anti-integrin αvβ6 antibody or the anti-integrin αvβ6 antibody-producing B cell, for example, a substance that specifically binds to the anti-integrin αvβ6 antibody or the anti-integrin αvβ6 antibody-producing B cell (hereinafter collectively referred to as "the substance that specifically binds to the anti-integrin αvβ6 antibody") may be used. The substance that specifically binds to the anti-integrin αvβ6 antibody is a substance that specifically binds to the anti-integrin αvβ6 antibody or the antigen receptor of the anti-integrin αvβ6 antibody-producing B cell to inhibit the binding between the anti-integrin αvβ6 antibody or the antigen receptor of the anti-integrin αvβ6 antibody-producing B cell and endogenous integrin αvβ6.

Examples of the substance that specifically binds to the anti-integrin αvβ6 antibody include, but not limited to, antigens for the antibody or the B cell, i.e., integrin αvβ6 or fragments thereof, antigen-like proteins or fragments thereof, peptides that specifically bind to anti-integrin αvβ6 antibodies, and compounds that specifically bind to anti-integrin αvβ6 antibodies.

In order to remove the anti-integrin αvβ6 antibody or the anti-integrin αvβ6 antibody-producing B cell, for example, a solid carrier on which the substance that specifically binds to the anti-integrin αvβ6 antibody is immobilized may be used. Examples of the solid carrier include, but not limited to, beads, fibers, membranes, hollow fiber membranes, gels, films, and their composite shapes, formed from carbohydrate materials such as agarose, dextran, cellulose, activated carbon, etc.; synthetic polymers such as polymethacrylate, polystyrene, polyacrylamide, polyamide, polysulfone, polyethylene, polyvinyl alcohol, polypropylene, polyacrylonitrile, polyethylene phthalate, polyester, styrene-divinylbenzene copolymer, ethylene-vinyl alcohol copolymer, etc.; inorganic materials such as silica gel, glass, metal, etc.; and composite materials with metals added. Commercially available solid carriers may be used. The substance may be immobilized on the solid carrier by a method known in the art, for example, by physical adsorption, covalent bonding, ionic bonding, chelate bonding, or avidin-biotin bonding. For the covalent bonding, for example, an active group such as cyanate ester, NHS (N-hydroxysuccinimide) ester, aldehyde, formyl, azlactone, CDI (carbonyldiimidazole), sulfhydryl, carbonyl, carboxyl, active hydrogen, epoxy, EAH (epoxy-activated hexahydrophthalic anhydride), ECH (epoxy-activated cyclohexane), thiopropyl, or activated thiol may be utilized. For the avidin-biotin bonding, for example, an avidin-like substance or a modified avidin, such as streptavidin, deglycosylated avidin [Neutravidin (registered trademark)], reversible avidin (Switchavidin), monomeric avidin [SAvPhire (registered trademark) monomeric streptavidin], mushroom-derived avidin-like protein [Tamavidin (registered trademark) 2-REV], genetically modified streptavidin, or genetically modified avidin may be used in place of avidin. Furthermore, the solid carrier on which the substance that specifically binds to the anti-integrin αvβ6 antibody is immobilized may be packed in a column or a bag such as a blood bag.

The removal of the anti-integrin αvβ6 antibody or the anti-integrin αvβ6 antibody-producing B cell can be carried out by contacting a sample derived from the subject, for example the subject's blood (e.g., whole blood, serum or plasma, preferably plasma), with the substance that specifically binds to the anti-integrin αvβ6 antibody. Preferably, the removal of the anti-integrin αvβ6 antibody or the anti-integrin αvβ6 antibody-producing B cell may be carried out by contacting a sample derived from the subject with a solid carrier on which the substance that specifically binds to the anti-integrin αvβ6 antibody is immobilized. For example, a column packed with the solid carrier on which the substance that specifically binds to the anti-integrin αvβ6 antibody is immobilized may be used to adsorb the anti-integrin αvβ6 antibody or the anti-integrin αvβ6 antibody-producing B cell in the subject's blood, and thereby the anti-integrin αvβ6 antibody or the anti-integrin αvβ6 antibody-producing B cell may be removed. The blood after the removal of the anti-integrin αvβ6 antibody or the anti-integrin αvβ6 antibody-producing B cell is preferably returned to the subject.

Thus, one aspect of the therapeutic method of the present invention provides apheresis for the treatment of ulcerative colitis or primary sclerosing cholangitis, the apheresis comprising drawing blood from a subject, treating the blood with the column, and then returning the blood to the subject.

In addition, another aspect of the present invention provides a method for removing the anti-integrin αvβ6 antibody or the anti-integrin αvβ6 antibody-producing B cell from a sample derived from a subject, the method comprising contacting the sample with the substance that specifically binds to the anti-integrin αvβ6 antibody.

### <5. Fragment or entirety of integrin αvβ6>

In the present invention, the origin of a fragment or an entirety of integrin αvβ6 is not particularly limited. The fragment or entirety of integrin αvβ6 is preferably from the same species as the subject. The nucleotide sequence information of genes encoding the αv chain and β6 chain of integrin from mammalian species including human, and the amino acid sequence information of each chain are available from known databases (GenBank, etc.). Specifically, the amino acid sequence of a preproprotein of human integrin αv chain isoform 1 is registered under GenBank Accession Number NP_002201.2 and is shown in SEQ ID NO:1. The amino acid sequence of a precursor of human integrin β6 chain is registered under GenBank Accession Number NP_000879.2 and is shown in SEQ ID NO:2. Similarly, the amino acid sequence information of integrin αv chains and β6 chains from various mammals other than a human are available from known databases (GenBank, etc.). The integrin αvβ6 may be composed of an αv chain and a β6 chain comprising amino acid sequences containing post-translational modification to one or both of the amino acid sequences of the αv chain and the β6 chain registered in a database. For example, the partial sequence from positions 1 to 30 in the amino acid sequence of SEQ ID NO:1 is a signal peptide sequence, and the amino acid sequence of the mature polypeptide of human integrin αv chain comprises the sequence from positions 31 to 1048 in the amino acid sequence of SEQ ID NO:1. Similarly, the partial sequence from positions 1 to 21 in the amino acid sequence of SEQ ID NO:2 is a signal peptide sequence, and the amino acid sequence of the mature polypeptide of the human integrin β6 chain comprises the sequence from positions 22 to 788 in the amino acid sequence of SEQ ID NO:2. In the amino acid sequence of the human integrin αv chain shown by SEQ ID NO:1, positions 31 to 992 correspond to an extracellular domain, positions 993 to 1016 correspond to a transmembrane domain, and positions 1017 to 1048 correspond to an intracellular domain. In the amino acid sequence of the human integrin β6 chain shown by SEQ ID NO:2, positions 22 to 707 correspond to an extracellular domain, positions 708 to 730 correspond to a transmembrane domain, and positions 731 to 788 correspond to an intracellular domain.

As used herein, unless otherwise specified, the fragment and the entirety of integrin αvβ6 are collectively referred to as "integrin αvβ6". Further, unless otherwise specified, the integrin αvβ6 in the present invention is not limited to the native form comprising a mature or immature amino acid sequence, and may be a mutant in a form equivalent to native integrin αvβ6.

The integrin αvβ6 is not limited to a form comprising the full lengths of both native or mutant α chain and β chain comprising mature or immature amino acid sequences (i.e., the entirety of integrin αvβ6), and may be in the form of a fragment of integrin αvβ6.

The fragment or entirety of integrin αvβ6 may be a fragment or entirety of integrin αvβ6 having another peptide attached to each chain thereof (e.g., attached to the C-terminus), or a fragment or entirety of integrin αvβ6 having biotin attached thereto, or an entirety of integrin αvβ6 having another peptide attached to each chain thereof (e.g., attached to the C-terminus) and biotin attached thereto. Examples of another peptide include, but not limited to, coiled-coil sequences (e.g., acidic or basic tail sequences), and tag (or label) sequences. The other peptide is used, for example, in production of the fragment or entirety of integrin αvβ6, for the purpose of facilitating dimerization, purification, or immobilization on carries. The other peptide may be added via a linker sequence. The linker sequence can be appropriately selected by those skilled in the art. Examples of the linker sequence include, but not limited to, glycine or serine-containing peptide linkers consisting of about 1 to 10 amino acids, such as GGGGSGGGGS, and glycine or serine-containing peptide linkers consisting of about 2 to 5 amino acids, such as GGGGS.

The fragment of integrin αvβ6 may be an integrin dimer comprising an αv chain and a β6 chain in which at least one of the αv chain and β6 chain constituting the integrin dimer is shorter than the α or β chain of the mature or immature, native or mutant integrin αvβ6. For example, the integrin αvβ6 fragment may be an integrin αvβ6 fragment comprising the extracellular domain of the αv chain or β6 chain. Thus, specific examples of the fragment of integrin αvβ6 include dimers comprising an αv chain and a β6 chain, wherein the αv chain comprises the partial sequence from Phe at position 31 to Val at position 992 of the αv chain amino acid sequence shown in SEQ ID NO:1 as the αv chain, and/or the β6 chain comprises the partial sequence from Gly at position 22 to Asn at position 707 of the β6 chain amino acid sequence shown in SEQ ID NO:2. The αv or β6 chain fragment may include a signal sequence. Therefore, another example of the fragment of integrin αvβ6 is a dimer comprising an αv chain having a partial sequence from Met at position 1 to Val at position 992 of the amino acid sequence of the αv chain shown by SEQ ID NO: 1, and/or a β6 chain having a partial sequence from Met at position 1 to Asn at position 707 of the amino acid sequence of the β6 chain shown by SEQ ID NO: 2. The fragment of integrin αvβ6 preferably forms a dimer, and more preferably has binding activity to an extracellular matrix protein such as laminin or fibronectin. The binding activity to an extracellular matrix protein of the integrin αvβ6 fragment can be determined by, for example, ELISA.

The fact that the entirety or fragment of integrin αvβ6 forms a dimer can be confirmed, for example, by detection of a band corresponding to the molecular weight of the dimer when the entirety or fragment of integrin αvβ6 is subjected to SDS-PAGE in the absence of 2-mercaptoethanol and disappearance of the band corresponding to the molecular weight of the dimer when the entirety or fragment of integrin αvβ6 is subjected to SDS-PAGE in the presence of 2-mercaptoethanol.

An example of commercially available integrin αvβ6 is recombinant human integrin αvβ6 (R&D Systems, Minnesota, USA, product number 3817-AV). This recombinant human integrin αvβ6 is a dimer of an αv chain consisting of the partial sequence from Phe at position 31 to Val at position 992 of the αv chain amino acid sequence shown in SEQ ID NO:1, and a linker sequence and an acidic tail sequence added to the C-terminus, and a β6 chain consisting of the partial sequence from Gly at position 22 to Asn at position 707 of the β6 chain amino acid sequence shown in SEQ ID NO:2, and a linker sequence and a basic tail sequence added to the C-terminus.

A more specific embodiment of the αv chain constituting the entirety or fragment of integrin αvβ6 is a polypeptide selected from the group consisting of:
(I) a polypeptide comprising the amino acid sequence shown in SEQ ID NO:1 or a partial sequence of the amino acid sequence shown in SEQ ID NO:1 from Phe at position 31 to Thr at position 1048;
(II) a polypeptide functionally equivalent to the polypeptide of (I), comprising a partial sequence of the amino acid sequence shown in SEQ ID NO:1;
(III) a polypeptide functionally equivalent to the polypeptide of (I), comprising an amino acid sequence having 85% or more sequence identity with the amino acid sequence shown in SEQ ID NO:1 or a partial sequence thereof; and
(IV) a polypeptide functionally equivalent to the polypeptide of (I), comprising an amino acid sequence in which one or more amino acids are substituted, deleted, and/or added in the amino acid sequence shown in SEQ ID NO:1 or a partial sequence thereof.

The polypeptides (I) to (IV) may comprise additional amino acid sequences as well as the amino acid sequences or partial sequences defined in (I) to (IV). The additional amino acid sequence is added to at least one of the N-terminus and C-terminus, preferably to the C-terminus, of the amino acid sequences or partial sequences defined in (I) to (IV).

In (II), (III) and (IV) as described above, an example of the polypeptide functionally equivalent to the polypeptide of (I) is a polypeptide capable of forming a dimer with an integrin β6 chain (particularly preferably, a polypeptide chain consisting of the amino acid sequence shown in SEQ ID NO:2, a polypeptide chain consisting of the partial sequence from Gly at position 22 to Cys at position 788 of the amino acid sequence shown in SEQ ID NO:2, or a polypeptide chain consisting of the partial sequence from Gly at position 22 to Asn at position 707 of the amino acid sequence shown in SEQ ID NO:2), wherein the formed dimer has the ability to bind to an extracellular matrix protein such as laminin or fibronectin to which native integrin αvβ6 or commercially available integrin αvβ6 can bind.

The partial sequence in (II) as described above includes the partial sequence from Phe at position 31 to Val at position 992 of the amino acid sequence shown in SEQ ID NO:1. The partial sequences in (III) and (IV) aa described above include the partial sequence from Phe at position 31 to Thr at position 1048 of the amino acid sequence shown in SEQ ID NO:1, and the partial sequence from Phe at position 31 to Val at position 992 of the amino acid sequence shown in SEQ ID NO:1.

The sequence identity in (III) as described above is preferably 90% or more, more preferably 95% or more, even more preferably 96% or more, particularly preferably 97% or more, and most preferably 98% or more or 99% or more.

In (IV) as described above, the term "one or more" means, for example 1 to 100, preferably 1 to 50, preferably 1 to 30, preferably 1 to 20, preferably 1 to 15, preferably 1 to 10, preferably 1 to 5, preferably 1 to 4, preferably 1 to 3, preferably 1 to 2, or preferably 1.

A more specific embodiment of the β6 chain constituting the entirety or fragment of integrin αvβ6 is a polypeptide selected from the group consisting of:
(V) a polypeptide comprising the amino acid sequence shown in SEQ ID NO:2 or a partial sequence of the amino acid sequence shown in SEQ ID NO:2 from Gly at position 22 to Cys at position 788;
(VI) a polypeptide functionally equivalent to the polypeptide of (V), comprising a partial sequence of the amino acid sequence shown in SEQ ID NO:2;
(VII) a polypeptide functionally equivalent to the polypeptide of (V), comprising an amino acid sequence having 85% or more sequence identity with the amino acid sequence shown in SEQ ID NO:2 or a partial sequence thereof; and
(VIII) a polypeptide functionally equivalent to the polypeptide of (V), comprising an amino acid sequence in which one or more amino acids are substituted, deleted, and/or added in the amino acid sequence shown in SEQ ID NO:2 or a partial sequence thereof.

The polypeptides (V) to (VIII) may comprise additional amino acid sequences as well as the amino acid sequences or partial sequences defined in (V) to (VIII). The additional amino acid sequence is added to at least one of the N-terminus and C-terminus, preferably to the C-terminus, of the amino acid sequences or partial sequences defined in (V) to (VIII).

In (VI), (VII) and (VIII) as described above, an example of the polypeptide functionally equivalent to the polypeptide of (V) is a polypeptide capable of forming a dimer with an integrin αv chain (particularly preferably, a polypeptide chain consisting of the amino acid sequence shown in SEQ ID NO:1, a polypeptide chain consisting of the partial sequence from Phe at position 31 to Thr at position 1048 of the amino acid sequence shown in SEQ ID NO:1, or a polypeptide chain consisting of the partial sequence from Phe at position 31 to Val at position 992 of the amino acid sequence shown in SEQ ID NO:1), wherein the formed dimer has the ability to bind to an extracellular matrix protein such as laminin or fibronectin to which native integrin αvβ6 or commercially available integrin αvβ6 can bind.

The partial sequence in (VI) as described above includes the partial sequence from Gly at position 22 to Asn at position 707 of the amino acid sequence shown in SEQ ID NO:2. The partial sequences in (VII) and (VIII) as described above include the partial sequence from Gly at position 22 to Cys at position 788 of the amino acid sequence shown in SEQ ID NO:2, and the partial sequence from Gly at position 22 to Asn at position 707 of the amino acid sequence shown in SEQ ID NO:2.

The sequence identity in (VII) as described above is preferably 90% or more, more preferably 95% or more, even more preferably 96% or more, particularly preferably 97% or more, and most preferably 98% or more or 99% or more.

In (VIII) as described above, the term "one or more" means, for example 1 to 100, preferably 1 to 50, preferably 1 to 30, preferably 1 to 20, preferably 1 to 15, preferably 1 to 10, preferably 1 to 5, preferably 1 to 4, preferably 1 to 3, preferably 1 to 2, or preferably 1.

In (III) and (VII) as described above, the amino acid sequence identity can be determined using a method well known to those skilled in the art, for example, sequence analysis software. Examples of the sequence analysis software include, but not limited to, blastp program of BLAST algorithm, and fasta program of FASTA algorithm.

### <6. System for treating ulcerative colitis or primary sclerosing cholangitis>

The system of the present invention is a system for treating ulcerative colitis or primary sclerosing cholangitis, comprising a means for removing an anti-integrin αvβ6 antibody or an anti-integrin αvβ6 antibody-producing B cell that produces the anti-integrin αvβ6 antibody, wherein the anti-integrin αvβ6 antibody has activity competing with fibronectin for binding to integrin αvβ6 and is specifically produced in a subject suffering from ulcerative colitis or primary sclerosing cholangitis.

As used herein, the "system" includes a single device, a combination of devices, and a treatment room.

The "means" for removing the anti-integrin αvβ6 antibody or the anti-integrin αvβ6 antibody-producing B cell may be any means as long as the anti-integrin αvβ6 antibody or the anti-integrin αvβ6 antibody-producing B cell is removed, and is not particularly limited. For example, the means may comprise a substance that removes the anti-integrin αvβ6 antibody or the anti-integrin αvβ6 antibody-producing B cell.

An example of the substance that removes the anti-integrin αvβ6 antibody or the anti-integrin αvβ6 antibody-producing B cell is the substance that specifically binds to the anti-integrin αvβ6 antibody as described above. For example, the means may be a device for adsorbing the anti-integrin αvβ6 antibody or the anti-integrin αvβ6 antibody-producing B cell.

The substance that specifically binds to the anti-integrin αvβ6 antibody may be immobilized on a solid carrier. The solid carrier on which the substance that specifically binds to the anti-integrin αvβ6 antibody is immobilized may further be packed into a column. The amount of the solid carrier packed in the column, the amount of the immobilized substance that specifically binds to the anti-integrin αvβ6 antibody, and the size of the column are not particularly limited, and can be appropriately selected by those skilled in the art. Examples of the substance that specifically binds to the anti-integrin αvβ6 antibody include, but not limited to, fragments of integrin αvβ6 and the entirety of integrin αvβ6.

Therefore, an example of the system of the present invention may include a column for treatment of ulcerative colitis or primary sclerosing cholangitis, the column carrying the substance that specifically binds to the anti-integrin αvβ6 antibody, thereby being able to adsorb the anti-integrin αvβ6 antibody or the anti-integrin αvβ6 antibody-producing B cell. The column may be a column containing a solid carrier on which the substance that specifically binds to the anti-integrin αvβ6 antibody is immobilized. The column may be regenerated after use by detaching the adsorbed anti-integrin αvβ6 antibody or the anti-integrin αvβ6 antibody-producing B cell. The column may preferably be a column for apheresis. For example, the system of the present invention may be a system for apheresis.

For example, the column for treatment may be a column containing a carrier on which integrin αvβ6 or a fragment thereof is immobilized. The carrier or column preferably specifically adsorbs an anti-integrin αvβ6 antibody comprising an RGD peptide sequence or a similar sequence to the RGD peptide in a heavy or light chain CDR, more preferably in heavy chain CDR2 or CDR3, and even more preferably in heavy chain CDR3, or a B cell that produces the antibody. The carrier or column preferably adsorbs an anti-integrin αvβ6 antibody or a B cell producing the antibody having a binding dissociation constant (KD value) for integrin αvβ6 or a fragment thereof of 100 nM or less, more preferably 50 nM or less, and particularly preferably 25 nM or less. In other words, the carrier or column preferably adsorbs an anti-integrin αvβ6 antibody or a B cell producing the antibody with a KD value of 100 nM or less, more preferably 50 nM or less, and particularly preferably 25 nM or less. On the carrier or column, preferably, integrin αvβ6 may be immobilized in an amount equal to or greater than twice the weight of the antibody to be captured, or a fragment of integrin αvβ6 may be immobilized in an amount equal to or greater than a value obtained by multiplying twice the weight of the antibody to be captured by a proportion of the molecular weight of the fragment to the molecular weight of the entirety of integrin αvβ6. More preferably, the carrier or column adsorbs an anti-integrin αvβ6 antibody or a B cell producing the antibody at an adsorption rate of 50% or more for integrin αvβ6 immobilized in an amount equal to or greater than twice the weight of the antibody to be captured, or for a fragment of integrin αvβ6 immobilized in an amount equal to or greater than a value obtained by multiplying twice the weight of the antibody to be captured by a proportion of the molecular weight of the fragment to the molecular weight of the entirety of integrin αvβ6. Furthermore, the column preferably contains a carrier on which the entirety of integrin αvβ6 or a fragment thereof is immobilized in an amount such that the integrin αvβ6 protein is at least twice the weight of the captured antibody, or the αvβ6 protein fragment is in an amount equal to or greater than a value obtained by multiplying twice the weight of the captured antibody by a proportion of the molecular weight of the fragment to the molecular weight of the entirety of integrin αvβ6. As used herein, the term "adsorption rate" refers to a proportion of the amount of antigen-bound antibody to the amount of antibody added in an antigen-antibody reaction system. For example, an antibody is added to a reaction system containing the carrier or column, and then the amount of free antibody (the amount of antibody not captured by the carrier or column) is measured after the reaction, and the proportion of the amount of captured antibody to the amount of antibody added to the reaction system is calculated.

Another example of the system of the present invention is a device for treating ulcerative colitis or primary sclerosing cholangitis, which may comprise one or more devices including the column. For example, the system may comprise, in addition to the column, one or more devices selected from the group consisting of a device for collecting blood from a subject, a device for returning blood to a subject, a pump for circulating blood, a plasma separator (e.g., a membrane-type plasma separator, etc.), a filter for removing foreign matters, fine particles, air bubbles, etc. from blood, a circuit for circulating blood (a blood supply line, a blood return line, etc.), a fluid replacement line, etc.

Further example of the system of the present invention is a treatment room for treating ulcerative colitis or primary sclerosing cholangitis, comprising a means for removing the anti-integrin αvβ6 antibody or the anti-integrin αvβ6 antibody-producing B cell. As used herein, the term "treatment room" refers to a section for accommodating a subject and treating the subject for the treatment of ulcerative colitis or primary sclerosing cholangitis, and is preferably an independent room. The treatment room preferably comprises the substance that specifically binds to the anti-integrin αvβ6 antibody. More preferably, the treatment room comprises the column or device. The treatment room may further comprise a treatment table, etc.

The devices and components for the system of the present invention may be controlled by computer.

The anti-integrin αvβ6 antibody, the anti-integrin αvβ6 antibody-producing B cell, the substance that specifically binds to the anti-integrin αvβ6 antibody, and the solid carrier are as described in the above section "4. Method for treating ulcerative colitis or primary sclerosing cholangitis."

### <7. Kit for treatment of ulcerative colitis or primary sclerosing cholangitis>

The present invention further provides a kit for treating ulcerative colitis or primary sclerosing cholangitis, comprising the substance that specifically binds to the anti-integrin αvβ6 antibody, the solid carrier on which the substance that specifically binds to the anti-integrin αvβ6 antibody is immobilized, or the column carrying the substance that specifically binds to the anti-integrin αvβ6 antibody. The substance that specifically binds to the anti-integrin αvβ6 antibody, the solid carrier, and the column are as described in the above section "4. Method for treating ulcerative colitis or primary sclerosing cholangitis."

### <8. Animal model for ulcerative colitis or primary sclerosing cholangitis>

The present invention further provides animal models of ulcerative colitis or primary sclerosing cholangitis, and methods for producing the same. The animal may be any non-human animal, preferably a non-human mammal. Examples of the animal include, but not limited to mice, rats, guinea pigs, rabbits, monkeys, goats, dogs, hamsters, ferrets, and pigs.

In one aspect, the present invention provides a method for producing an animal model of primary sclerosing cholangitis, the method comprising immunizing an animal with integrin αvβ6. Anti-integrin αvβ6 autoantibodies are produced in the immunized animals, resulting in the development of the above-mentioned symptoms.

In the method for producing an animal model of primary sclerosing cholangitis, integrin αvβ6 may be administered to the animal preferably together with an adjuvant. In the method for producing an animal model of primary sclerosing cholangitis, the frequency of immunizations is not particularly limited. Integrin αvβ6 may be administered preferably two or more times, for example, two to five times at appropriate intervals, for example, three times every two weeks or three times every four weeks. Administration of integrin αvβ6 is, for example, by injection, for example subcutaneous injection in the back. The administration route, dosage, frequency and number of integrin αvβ6 can be appropriately determined by those skilled in the art. For example, a dose of 50 µg/200 µl PBS of integrin αvβ6 may be mixed with 200 µl of CFA and then, administered by subcutaneous injection into the back of the animal.

In another aspect, a method for producing an animal model of primary sclerosing cholangitis, comprising knocking out the integrin αvβ6 gene in an animal is provided. Gene knockout can be achieved by a known method such as genome editing or a method using ES cells in which the target gene has been disrupted. According to the present invention, it has been revealed that the binding of anti-integrin αvβ6 autoantibodies to integrin αvβ6 present in the body of an animal, i.e., the inhibition or loss of function of integrin αvβ6 due to the binding of autoantibodies is involved in the formation of the pathological condition of primary sclerosing cholangitis. In the present invention, the integrin αvβ6 gene was knocked out to cause deficiency of integrin αvβ6 itself, and thereby the onset of primary sclerosing cholangitis was successfully induced.

In another aspect, provided is a method for producing an animal model of ulcerative colitis or primary sclerosing cholangitis, the method comprising administering an anti-integrin αvβ6 antibody derived from a patient with ulcerative colitis or primary sclerosing cholangitis to an animal. The patient is an animal, preferably a mammal, and more preferably a human. Examples of the antibody include antiserum (polyclonal antibody) and a monoclonal antibody. The antiserum may be a serum derived from a patient with ulcerative colitis or primary sclerosing cholangitis. Preferably, a monoclonal antibody is administered. The monoclonal antibodies may be prepared from a serum derived from a patient with ulcerative colitis or primary sclerosing cholangitis by a known method. For example, a hybridoma method is used.

The administration method, administration route, dosage, number of administrations, administration frequency, and administration intervals of the antibody can be appropriately determined by those skilled in the art. For example, the serum from the patient may be administered to an animal by injection one or more times. For example, 1-3 ml per animal of the patient serum may be administered 1-3 times at an appropriate interval. For example, 2 ml per animal of the patient serum may be administered by subcutaneous injection in the animal's back. In this case, the symptoms of ulcerative colitis or primary sclerosing cholangitis appear at the latest about 24 hours after administration. For example, an anti-integrin αvβ6 monoclonal antibody may be administered to an animal by injection, preferably two or more times. For example, 1 mg to 20 mg per animal of the monoclonal antibody may be administered by injection 1 to 3 times a week, for example, for 1 to 3 weeks. For example, 10 mg per animal of the anti-integrin αvβ6 antibody may be administered by subcutaneous injection in the back twice a week for three weeks (total six times). In this case, the symptoms of ulcerative colitis or primary sclerosing cholangitis appear at the latest about 48 hours after the last administration.

The antibody preferably comprises an RGD peptide sequence or a similar sequence thereto in CDR2 or CDR3, more preferably in heavy chain CDR2 or CDR3, and particularly preferably in heavy chain CDR3. The similar sequences to the RGD peptide are described in section <2. Anti-integrin αvβ6 antibody>.

The monoclonal antibody is, for example, selected from:
(a) a polypeptide that specifically binds to integrin αvβ6 or a fragment thereof, the polypeptide comprising three heavy chain CDRs respectively having sequences shown by SEQ ID NOs: 11 to 13 and three light chain CDRs respectively having sequences shown by SEQ ID NOs: 14 to 16, and comprising a heavy chain variable region having at least 85%, 90%, 95%, 96%, 97%, 98% or 99% sequence identity with the amino acid sequence shown by SEQ ID NO: 83 and a light chain variable region having at least 85%, 90%, 95%, 96%, 97%, 98% or 99% sequence identity with the amino acid sequence shown by SEQ ID NO: 84;
(b) a polypeptide that specifically binds to integrin αvβ6 or a fragment thereof, the polypeptide comprising three heavy chain CDRs respectively having sequences shown by SEQ ID NO: 17 to 19 and three light chain CDRs respectively having sequences shown by SEQ ID NO: 20 to 22, and comprising a heavy chain variable region having at least 85%, 90%, 95%, 96%, 97%, 98% or 99% sequence identity with the amino acid sequence shown by SEQ ID NO: 85 and a light chain variable region having at least 85%, 90%, 95%, 96%, 97%, 98% or 99% sequence identity with the amino acid sequence shown by SEQ ID NO: 86;
(c) a polypeptide that specifically binds to integrin αvβ6 or a fragment thereof, the polypeptide comprising three heavy chain CDRs respectively having sequences shown by SEQ ID NO: 23 to 25 and three light chain CDRs respectively having sequences shown by SEQ ID NO: 26 to 28, and comprising a heavy chain variable region having at least 85%, 90%, 95%, 96%, 97%, 98% or 99% sequence identity with the amino acid sequence shown by SEQ ID NO: 87 and a light chain variable region having at least 85%, 90%, 95%, 96%, 97%, 98% or 99% sequence identity with the amino acid sequence shown by SEQ ID NO: 88;
(d) a polypeptide that specifically binds to integrin αvβ6 or a fragment thereof, the polypeptide comprising three heavy chain CDRs respectively having sequences shown by SEQ ID NOs: 29 to 31 and three light chain CDRs respectively having sequences shown by SEQ ID NOs: 32 to 34, and comprising a heavy chain variable region having at least 85%, 90%, 95%, 96%, 97%, 98% or 99% sequence identity with the amino acid sequence shown by SEQ ID NO: 89 and a light chain variable region having at least 85%, 90%, 95%, 96%, 97%, 98% or 99% sequence identity with the amino acid sequence shown by SEQ ID NO: 90;
(e) a polypeptide that specifically binds to integrin αvβ6 or a fragment thereof, the polypeptide comprising three heavy chain CDRs respectively having sequences shown by SEQ ID NO: 35 to 37 and three light chain CDRs respectively having sequences shown by SEQ ID NO: 38 to 40, and comprising a heavy chain variable region having at least 85%, 90%, 95%, 96%, 97%, 98% or 99% sequence identity with the amino acid sequence shown by SEQ ID NO: 91 and a light chain variable region having at least 85%, 90%, 95%, 96%, 97%, 98% or 99% sequence identity with the amino acid sequence shown by SEQ ID NO: 92;
(f) a polypeptide that specifically binds to integrin αvβ6 or a fragment thereof, the polypeptide comprising three heavy chain CDRs respectively having sequences shown by SEQ ID NO: 41 to 43 and three light chain CDRs respectively having sequences shown by SEQ ID NO: 50 to 52, and comprising a heavy chain variable region having at least 85%, 90%, 95%, 96%, 97%, 98% or 99% sequence identity with the amino acid sequence shown by SEQ ID NO: 93 and a light chain variable region having at least 85%, 90%, 95%, 96%, 97%, 98% or 99% sequence identity with the amino acid sequence shown by SEQ ID NO: 94;
(g) a polypeptide that specifically binds to integrin αvβ6 or a fragment thereof, the polypeptide comprising three heavy chain CDRs respectively having sequences shown by SEQ ID NO: 47 to 49 and three light chain CDRs respectively having sequences shown by SEQ ID NO: 44 to 46, and comprising a heavy chain variable region having at least 85%, 90%, 95%, 96%, 97%, 98% or 99% sequence identity with the amino acid sequence shown by SEQ ID NO: 95 and a light chain variable region having at least 85%, 90%, 95%, 96%, 97%, 98% or 99% sequence identity with the amino acid sequence shown by SEQ ID NO: 96
(h) a polypeptide that specifically binds to integrin αvβ6 or a fragment thereof, the polypeptide comprising three heavy chain CDRs respectively having sequences shown by SEQ ID NOs: 53 to 55 and three light chain CDRs respectively having sequences shown by SEQ ID NOs: 56 to 58, and comprising a heavy chain variable region having at least 85%, 90%, 95%, 96%, 97%, 98% or 99% sequence identity with the amino acid sequence shown by SEQ ID NO: 97 and a light chain variable region having at least 85%, 90%, 95%, 96%, 97%, 98% or 99% sequence identity with the amino acid sequence shown by SEQ ID NO: 98;
(i) a polypeptide that specifically binds to integrin αvβ6 or a fragment thereof, the polypeptide comprising three heavy chain CDRs respectively having sequences shown by SEQ ID NOs: 59 to 61 and three light chain CDRs respectively having sequences shown by SEQ ID NOs: 62 to 64, and comprising a heavy chain variable region having at least 85%, 90%, 95%, 96%, 97%, 98% or 99% sequence identity with the amino acid sequence shown by SEQ ID NO: 99 and a light chain variable region having at least 85%, 90%, 95%, 96%, 97%, 98% or 99% sequence identity with the amino acid sequence shown by SEQ ID NO: 100;
(j) a polypeptide that specifically binds to integrin αvβ6 or a fragment thereof, the polypeptide comprising three heavy chain CDRs respectively having sequences shown by SEQ ID NOs: 65 to 67 and three light chain CDRs respectively having sequences shown by SEQ ID NOs: 68 to 70, and comprising a heavy chain variable region having at least 85%, 90%, 95%, 96%, 97%, 98% or 99% sequence identity with the amino acid sequence shown by SEQ ID NO: 101 and a light chain variable region having at least 85%, 90%, 95%, 96%, 97%, 98% or 99% sequence identity with the amino acid sequence shown by SEQ ID NO: 102;
(k) a polypeptide that specifically binds to integrin αvβ6 or a fragment thereof, the polypeptide comprising three heavy chain CDRs respectively having sequences shown by SEQ ID NOs: 71 to 73 and three light chain CDRs respectively having sequences shown by SEQ ID NOs: 74 to 76, and comprising a heavy chain variable region having at least 85%, 90%, 95%, 96%, 97%, 98% or 99% sequence identity with the amino acid sequence shown by SEQ ID NO: 103 and a light chain variable region having at least 85%, 90%, 95%, 96%, 97%, 98% or 99% sequence identity with the amino acid sequence shown by SEQ ID NO: 104; or
(l) a polypeptide that specifically binds to integrin αvβ6 or a fragment thereof, the polypeptide comprising three heavy chain CDRs respectively having sequences shown by SEQ ID NOs: 77 to 79 and three light chain CDRs respectively having sequences shown by SEQ ID NOs: 80 to 82, and comprising a heavy chain variable region having at least 85%, 90%, 95%, 96%, 97%, 98% or 99% sequence identity with the amino acid sequence shown by SEQ ID NO: 105 and a light chain variable region having at least 85%, 90%, 95%, 96%, 97%, 98% or 99% sequence identity with the amino acid sequence shown by SEQ ID NO: 106. Preferably a polypeptide selected from (a) to (i), (k) or (l) is used, more preferably a polypeptide selected from (a) to (i) or (k) is used, and even more preferably a polypeptide selected from (b) to (i) or (k) is used.

Preferably, an anti-integrin αvβ6 antibody derived from a patient with ulcerative colitis is used to prepare an animal model of ulcerative colitis, and an anti-integrin αvβ6 antibody derived from a patient with primary sclerosing cholangitis is used to prepare an animal model of primary sclerosing cholangitis. For example, a polypeptide selected from (a) to (j) as mentioned above, preferably a polypeptide selected from (a) to (i) as mentioned above, more preferably a polypeptide selected from (b) to (i) as mentioned above, even more preferably a polypeptide selected from (c) or (d) as mentioned above is used preferably for producing an animal model of ulcerative colitis. For example, a polypeptide selected from (k) or (l) as mentioned above, preferably polypeptide (k) as mentioned above is used preferably for producing an animal model of primary sclerosing cholangitis. However, because CDR3 is the most important site for antibody specificity (binding ability), when an antibody derived from an ulcerative colitis patient and an antibody derived from a primary sclerosing cholangitis patient have a common CDR3 sequence, the antibody derived from an ulcerative colitis patient or the antibody derived from a primary sclerosing cholangitis patient may be used to prepare both an animal model of ulcerative colitis and an animal model of primary sclerosing cholangitis.

The animal may receive dextran sodium sulfate (DSS) in advance. DSS is a drug known to induce enteritis. Since it is known that the expression of integrin αvβ6 increases during inflammation or healing, DSS is administered to the animal in advance to increase the amount of integrin αvβ6 in the animal body in advance and thereby increase the reaction of integrin αvβ6 with anti-integrin αvβ6 autoantibodies, and thus the incidence of the diseases can be increased. The amount of DSS to be administered can be determined appropriately by those skilled in the art, and is not particularly limited. Preferably, DSS in an amount sufficient to cause mild inflammation, for example, about 1% DSS may be administered via a water supply. DSS can be administered, for example, orally or by injection, preferably orally. The administration period of DSS is not particularly limited, and may be appropriately determined by those skilled in the art. For example, DSS may be administered for about one day to three weeks. After administration of DSS, drug holidays are preferably provided to allow inflammation to completely subside, and then the patient's serum or the monoclonal antibody is administered. The drug holidays are not particularly limited, and may be, for example, 3 weeks or more.

The animal model of ulcerative colitis produced by the method as described above has the symptoms of ulcerative colitis, in particular, cellular infiltration (e.g., submucosal cellular infiltration) in the large intestine, or crypt distortion. The animal model of primary sclerosing cholangitis produced by the method as described above has the symptoms of primary sclerosing cholangitis, in particular, partial loss of the bile duct, or cellular infiltration or fibrosis around the bile duct. The animal models thus obtained are ideal animal models for ulcerative colitis or primary sclerosing cholangitis, and the use of the models enables the development of various therapeutic drugs. Therefore, as a further aspect of the present invention, provided is a method for screening for a therapeutic or preventive drug for ulcerative colitis or primary sclerosing cholangitis, the method comprising administering a candidate substance for treating ulcerative colitis or primary sclerosing cholangitis to the model animal.

As described above, the inventors previously found that the presence of autoantibodies against integrin αvβ6 in patients is indicative of ulcerative colitis and primary sclerosing cholangitis (WO 2020/141608, Gastroenterology Vol. 160, No. 7, June 2021, Pages 2383-2394). For ulcerative colitis, many serological antibodies other than autoantibodies against integrin αvβ6 have been reported as indicators of ulcerative colitis. However, such serological antibodies are believed to be markers of abnormal immune response rather than direct effectors involved in the pathogenesis of ulcerative colitis (World J Gastroenterol, 2016 January 21, 22(3): 1304-1310). Before the present invention, autoantibodies against integrin αvβ6 were also recognized in the medical community as a marker of abnormal immune response, rather than a direct effector involved in the pathogenesis of ulcerative colitis.

### EXAMPLES

Hereinafter, the present invention is explained in more detail using Examples which the present invention is not limited to.

### <Example 1. Demonstration experiment regarding anti-integrin αvβ6 autoantibody specifically produced in PSC patient>

### 1. Inhibition of integrin αvβ6-fibronectin binding by antibody derived from PSC patient

Inhibition of the binding of integrin αvβ6 to fibronectin by antibodies (IgG: immunoglobulin G) derived from human PSC patients was investigated using a solid-phase binding assay.

### Methods

### (1) Preparation of human IgG

IgG was purified from the sera of PSC patients or controls (patients with cholangiocellular carcinoma, IgG4-associated sclerosing cholangitis, autoimmune hepatitis, primary cholestatic cholangitis, or other autoimmune diseases, or healthy people) using Ab-Rapid SpinN (P-013, ProteNova, Higashikagawa, Japan) according to the manufacturer's instructions. The purified IgG was dialyzed against phosphate-buffered saline (pH 7.2), concentrated by ultrafiltration using an Amicon Ultra filter (UFC805024; Millipore) to the same volume as the serum before purification, and stored at -20°C. The concentration of purified IgG was measured using a human IgG enzyme immunoassay kit (MK136; TaKaRa). The purity of the IgG fraction was determined by testing for IgA, IgA, IgE, and protein contaminants using a human IgA ELISA kit (E88-102; Bethyl Laboratories), a human IgM ELISA kit (E88-100; Bethyl Laboratories), a human IgE ELISA kit (E88-108; Bethyl Laboratories), and sodium dodecyl sulfate polyacrylamide gel electrophoresis with Coomassie brilliant blue staining, respectively.

### (2) Solid-phase integrin αvβ6 binding assay

A 96-well microtiter plate was coated with 150 µL/well of 2 µg/mL human integrin αvβ6 (ACROBiosystems, product no. IT6-H52E1) at 4°C overnight, blocked and then incubated with 120 µL of diluted IgG from the patients or controls (1:10 to 1:80) at room temperature for 60 minutes. After washing, the plate was incubated with 100 µL of 2 µg/mL fibronectin (FC010; MilliporeSigma, Burlington, Mass.) at room temperature for 60 minutes. After washing, an anti-fibronectin antibody (1:5000 dilution; ab2413; Abcam) was added to the plate and incubated at room temperature for 60 min. After washing, an HRP-labeled anti-rabbit antibody (1:10,000 dilution; A27036; ThermoFisher Scientific) was added to the plate and incubated at room temperature for 60 min. After washing, the plate was incubated with 3,3',5,5'-tetramethylbenzidine for 10 minutes, and the absorbance at 450 nm was measured to detect fibronectin bound to integrin αvβ6 on the solid phase. The assay was carried out in the presence of MgCl₂ (1 mM) and CaCl₂ (1 mM). The human integrin αvβ6 (ACROBiosystems, product number IT6-H52E1) is a heterodimeric protein comprising an α chain comprising an extracellular domain extending from Phe at position 31 to Val at position 992 in the amino acid sequence of the human integrin αv chain, and a linker sequence, an acidic tail sequence and a polyhistidine tag at the C-terminus, and a β chain comprising an extracellular domain extending from Gly at position 22 to Asn at position 707 in the amino acid sequence of the human integrin β6 chain, and a linker sequence and a basic tail sequence at the C-terminus.

To calculate a binding inhibition rate, blank wells were prepared by coating with integrin αvβ6 and incubating with fibronectin in the absence of IgG from the patients or controls, and the optical density (OD) at 450 nm was measured in the same manner as above. The binding inhibition rate (%) was calculated according to the following formula. Binding inhibition rate (%) = {[(OD value of blank well) - (OD value of well to which IgG was added)]/(OD value of blank well)} × 100

### Results

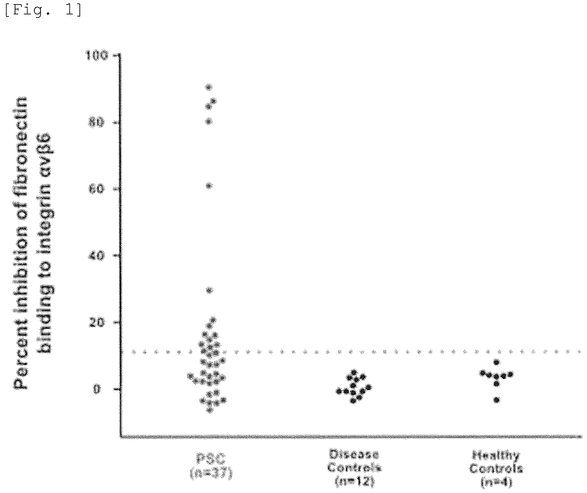

Results of the binding inhibition test using 1:10 diluted IgG are shown in Figure 1. In Figure 1, a cutoff OD value level is indicated by a dashed line. The cutoff OD value is calculated by adding a value three times as much as a standard deviation (SD) to the average value of control IgG. IgG from 15 of 37 PSC patients (40.5%) inhibited the binding of integrin αvβ6 to fibronectin (plotted as values above the cutoff OD value in FIG. 1). On the other hand, control IgG derived from patients with control diseases (n=12) did not inhibit the binding of integrin αvβ6 to fibronectin. Furthermore, IgG derived from healthy people (four people) did not inhibit the binding of integrin αvβ6 to fibronectin.

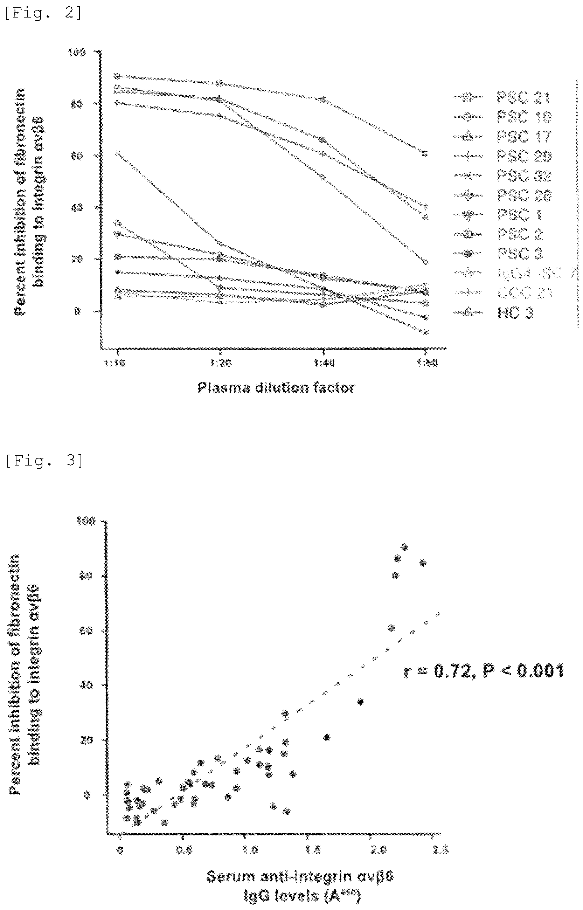

Figure 2 shows dose-dependent inhibition of binding of integrin αvβ6 to fibronectin by autoantibodies derived from patients with primary sclerosing cholangitis. The integrin αvβ6-fibronectin binding inhibitory activity of the PSC patient IgG was shown to be dose-dependent (Figure 2). The IgG from PSC patients (denoted as PSC21, PSC19, PSC17, PSC29, PSC32, PSC26, PSC1, PSC2, and PSC3 in Figure 2) containing anti-integrin αvβ6 antibodies dose-dependently inhibited the binding of integrin αvβ6 to fibronectin. In contrast, the IgG from the control patients with IgG4-related sclerosing cholangitis (denoted as IgG4-SC7 in Figure 2), and cholangiocellular carcinoma (denoted as CCC21 in Figure 2), and a healthy control (denoted as HC3 in Figure 2) did not show inhibitory activity.

Furthermore, Figure 3 shows a correlation between the titers of autoantibodies derived from patients with primary sclerosing cholangitis and the inhibitory activity of integrin αvβ6-fibronectin binding. The integrin αvβ6-fibronectin binding inhibitory activity of the PSC patient IgG was shown to correlate with the anti-integrin αvβ6 antibody titer (r=0.72, P<0.001) (Figure 3).

### 2. Binding of antibody derived from PSC patient to fibronectin motif binding site

Integrin αvβ6 is known to bind to a ligand such as fibronectin by recognizing a RGD sequence motif. The present inventors hypothesized that an anti-integrin αvβ6 antibody derived from a PSC patient exerts its inhibitory activity by targeting the RGD binding site of integrin αvβ6, and examined the binding site of the antibody.

### Method

A microtiter plate was coated with 100 µL/well of 2 µg/mL integrin αvβ6 overnight at 4°C, and then blocked. Peptide RGDS (SEQ ID NO: 9: Arg-Gly-Asp-Ser) or RGES (SEQ ID NO: 10: Arg-Gly-Glu-Ser) as RGD motif or RGE motif (control) was added together with 100 µL of patient-derived IgG dilution (1:100) to the plate, and the plate was incubated at room temperature for 60 minutes. After washing, 100 µl/well of an HRP-labeled anti-human IgG antibody (50,000-fold dilution; ab6759; Abcam) was added to the plate and the plate was incubated at room temperature for 60 minutes. After washing, the plate was incubated with 3,3',5,5'-tetramethylbenzidine for 7 minutes, and an absorbance density (OD value) at 450 nm was measured to detect human IgG bound to integrin αvβ6 on the solid phase.

### Results

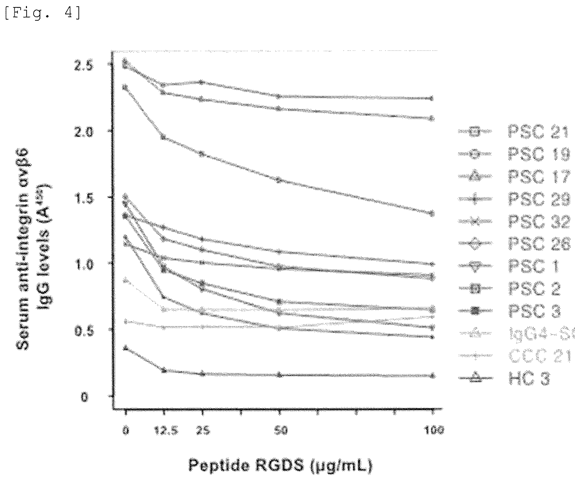

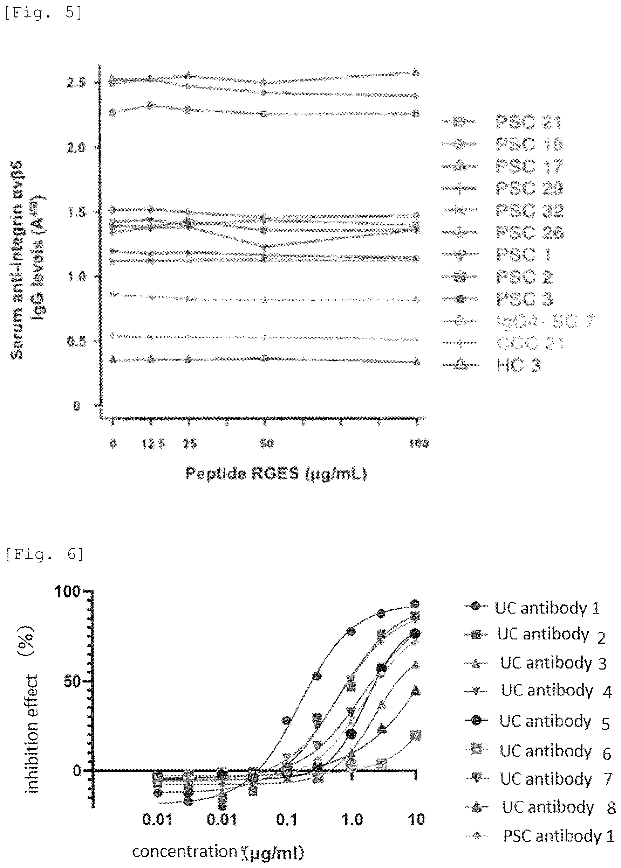

Figure 4 shows results of inhibition assay of binding of autoantibodies derived from patients with primary sclerosing cholangitis to integrin αvβ6 by addition of peptide RGDS (note that the notation used to identify each patient in Figure 4 is the same as in Figure 2). The peptide RGDS dose-dependently reduced the binding of PSC patient IgG to integrin αvβ6. On the other hand, Figure 5 shows results of inhibition assay of binding of autoantibodies derived from patients with primary sclerosing cholangitis to integrin αvβ6 by addition of peptide RGES (note that the notation for identifying each patient in Figure 5 is the same as in Figure 2). the peptide RGES did not inhibit the binding of PSC patient IgG to integrin αvβ6. Thus, it was demonstrated that the RGD peptide and the anti-integrin αvβ6 antibody compete for binding to the RGD motif-binding site on integrin αvβ6.

### <Example 2. Establishment of monoclonal anti-integrin αvβ6 antibody>

### 1. Establishment of monoclonal antibody

Monoclonal anti-integrin αvβ6 antibodies were established from peripheral blood of human patients with ulcerative colitis or primary sclerosing cholangitis by a standard method. Briefly, peripheral blood was obtained from patients with ulcerative colitis and primary sclerosing cholangitis, and immortalized by infection with EB virus, and clones producing anti-integrin αvβ6 antibodies were selected. The IgG DNA sequence of the clone was determined by sequencing, and introduced into a plasmid. The plasmid was then transfected into CHO cells to establish a monoclonal anti-integrin αvβ6 antibody. As a result, four monoclonal anti-integrin αvβ6 antibodies (referred to as UC antibody 1, UC antibody 2, UC antibody 3, and UC antibody 4) were established from one ulcerative colitis patient, and one monoclonal anti-integrin αvβ6 antibody (referred to as PSC antibody 1 and PSC antibody 2) was established each from two primary sclerosing cholangitis patients. Furthermore, four, one, and one monoclonal anti-integrin αvβ6 antibodies (referred to as UC antibodies 5 to 8, UC antibody 9, and UC antibody 10) were established each from three other patients with ulcerative colitis.

Sequence analysis of the obtained monoclonal antibodies revealed that their heavy chain CDR3 or CDR2 sequences contain an RGD motif or a similar sequence to RGD. In addition, another research group reported that the heavy chain CDR3 of monoclonal antibodies derived from UC patients contained an RGD motif or a sequence similar to RGD, such as RED, KGD, or SGD, and it has been shown that the antibodies containing such a sequence had high ability to bind to integrin αvβ6 (Nature Medicine volume 28, 766-779 (2022), J Exp Med (2023) 220 (4): e20220538.).

The heavy and light chain CDR sequences of the monoclonal antibodies thus obtained are shown below. Additionally, the amino acid and nucleotide sequences of the variable regions are shown. In the amino acid sequences of the variable regions, the CDR sequences are underlined and in bold.

**[Table 1-1]**

| Antibody | Amino acid sequence | | |
|---|---|---|---|
| UC antibody 1 | heavy chain CDR1 | GFTFSSYG | SEQ ID NO:11 |
| | heavy chain CDR2 | ISYDGINK | SEQ ID NO:12 |
| | heavy chain CDR3 | AKVIPRIRGSGDKAGIKDYYYYGMDV | SEQ ID NO:13 |
| | light chain CDR1 | QNLLHSNGYNY | SEQ ID NO:14 |
| | light chain CDR2 | LGS | SEQ ID NO:15 |
| | light chain CDR3 | MQALQTWT | SEQ ID NO:16 |
| UC antibody 2 | heavy chain CDR1 | GFTITNAW | SEQ ID NO:17 |
| | heavy chain CDR2 | SKSKTDGGTT | SEQ ID NO:18 |
| | heavy chain CDR3 | ATDRPLKLRGRDYNYYVMDV | SEQ ID NO:19 |
| | light chain CDR1 | QSIARS | SEQ ID NO:20 |
| | light chain CDR2 | AAS | SEQ ID NO:21 |
| | light chain CDR3 | QQSSSSPLT | SEQ ID NO:22 |
| UC antibody 3 | heavy chain CDR1 | GFTFSIYG | SEQ ID NO:23 |
| | heavy chain CDR2 | ISSDGTNQ | SEQ ID NO:24 |
| | heavy chain CDR3 | AKDRGRRGDSGWYRHFDY | SEQ ID NO:25 |
| | light chain CDR1 | SSNNGNNY | SEQ ID NO:26 |
| | light chain CDR2 | DNN | SEQ ID NO:27 |
| | light chain CDR3 | GTWDSSLSAVV | SEQ ID NO:28 |
| UC antibody 4 | heavy chain CDR1 | GYTFSSFG | SEQ ID NO:29 |
| | heavy chain CDR2 | ISAYNGNT | SEQ ID NO:30 |
| | heavy chain CDR3 | ARDRGFRGDTAMIKGGMDV | SEQ ID NO:31 |
| | light chain CDR1 | ALPKQY | SEQ ID NO:32 |
| | light chain CDR2 | KDS | SEQ ID NO:33 |
| | light chain CDR3 | SSTDSNSQRV | SEQ ID NO:34 |
| UC antibody 5 | heavy chain CDR1 | GHTFSSFG | SEQ ID NO:35 |
| | heavy chain CDR2 | ISAYNGNT | SEQ ID NO:36 |
| | heavy chain CDR3 | ARDRGFRGDTAMIKGGMDV | SEQ ID NO:37 |
| | light chain CDR1 | KLGDKY | SEQ ID NO:38 |
| | light chain CDR2 | QDS | SEQ ID NO:39 |
| | light chain CDR3 | QAWDSSTALV | SEQ ID NO:40 |
| UC antibody 6 | heavy chain CDR1 | GHTFSSFG | SEQ ID NO:41 |
| | heavy chain CDR2 | ISAYNGNT | SEQ ID NO:42 |
| | heavy chain CDR3 | ARDRGFRGDTAMIKGGMDV | SEQ ID NO:43 |
| | light chain CDR1 | SSTIGANND | SEQ ID NO:44 |
| | light chain CDR2 | GNK | SEQ ID NO:45 |
| | light chain CDR3 | QSYDSSLSDLYV | SEQ ID NO:46 |

**[Table 1-2]**

| | | | |
|---|---|---|---|
| UC antibody 7 | heavy chain CDR1 | GFTFSSYG | SEQ ID NO:47 |
| | heavy chain CDR2 | ISYDGINK | SEQ ID NO:48 |
| | heavy chain CDR3 | AKVIPRIRGSGDKAGIKDYYYYGMDV | SEQ ID NO:49 |
| | light chain CDR1 | QSVSSY | SEQ ID NO:50 |
| | light chain CDR2 | DAS | SEQ ID NO:51 |
| | light chain CDR3 | QQRSNWLT | SEQ ID NO:52 |
| UC antibody 8 | heavy chain CDR1 | GFTFSSYA | SEQ ID NO:53 |
| | heavy chain CDR2 | ISYDGINK | SEQ ID NO:54 |
| | heavy chain CDR3 | AKVIPRIRGSGDKAGIKDYYYYGMDV | SEQ ID NO:55 |
| | light chain CDR1 | QSVSSSY | SEQ ID NO:56 |
| | light chain CDR2 | GAS | SEQ ID NO:57 |
| | light chain CDR3 | QQYGSSVWT | SEQ ID NO:58 |
| UC antibody 9 | heavy chain CDR1 | GFTFSSYG | SEQ ID NO:59 |
| | heavy chain CDR2 | ISYDGINK | SEQ ID NO:60 |
| | heavy chain CDR3 | AKVIPRIRGSGDKAGIKDYYYYGMDV | SEQ ID NO:61 |
| | light chain CDR1 | SSNIGNNY | SEQ ID NO:62 |
| | light chain CDR2 | DNN | SEQ ID NO:63 |
| | light chain CDR3 | GTWDSSLSAVV | SEQ ID NO:64 |
| UC antibody 10 | heavy chain CDR1 | GYTLTELRI | SEQ ID NO:65 |
| | heavy chain CDR2 | DPEDGETIY | SEQ ID NO:66 |
| | heavy chain CDR3 | TTDLFAFVRGVRGAFDI | SEQ ID NO:67 |
| | light chain CDR1 | TITTYL | SEQ ID NO:68 |
| | light chain CDR2 | ASSLH | SEQ ID NO:69 |
| | light chain CDR3 | QQSYRTLWT | SEQ ID NO:70 |
| PSC antibody 1 | heavy chain CDR1 | GHTFSSFG | SEQ ID NO:71 |
| | heavy chain CDR2 | ISAYNGNT | SEQ ID NO:72 |
| | heavy chain CDR3 | ARDRGFRGDTAMIKGGMDV | SEQ ID NO:73 |
| | light chain CDR1 | SSDVGGYNY | SEQ ID NO:74 |
| | light chain CDR2 | DVS | SEQ ID NO:75 |
| | light chain CDR3 | SSYTSSSTYV | SEQ ID NO:76 |
| PSC antibody 2 | heavy chain CDR1 | GFDFSNYV | SEQ ID NO:77 |
| | heavy chain CDR2 | ITDRGDSR | SEQ ID NO:78 |
| | heavy chain CDR3 | AKDQTFAGDDPTVFDS | SEQ ID NO:79 |
| | light chain CDR1 | SSDIGGYNY | SEQ ID NO:80 |
| | light chain CDR2 | DVN | SEQ ID NO:81 |
| | light chain CDR3 | NSYTTSSTSV | SEQ ID NO:82 |

**[Table 2-1]**

| UC antibody 1 |
|---|
| Heavy chain variable region |
| QVQLVESGGGVVQPGRSLRLSCAASGFTFSSYGMHWVRQAPGKGLEWVAFISY DGINKYYADSVKGRFTISRDNSKDTLYLQMNSLRAEDTAVYYCAKVIPRIRGSG DKAGIKDYYYYGMDVWGQGTTVTVSS (SEQ ID NO:83) |
| Light chain variable region |
| DIVMTQSPLSLPVTPGEPASISCRSSQNLLHSNGYNYLDWYLQKPGQSPQLLIYL GSNRASGVPDRFSGSGSGTDFTLKISRVEAEDVGVYYCMQALQTWTFGQGTKV EIK (SEQ ID NO:84) |

| UC antibody 2 |
|---|
| Heavy chain variable region |
| EVQLVESGGGLVKPGGSLRVSCVVSGFTITNAWMSWVRQAPGKGLEWVGRSK SKTDGGTTDYIAPVKGRFTISRDDSKNTLYLEMNSLKTEDTAVYYCATDRPLKL RGRDYNYYVMDVWGQGTTVTVSS (SEQ ID NO:85) |
| Light chain variable region |
| DIQMTQSPSSLSASVGDRVTITCRASQSIARSLNWYQQKPGKAPNLLIYAASTL QSGVPSRFSGSGSGTDFTLTIGSLQPEDFATYSCQQSSSSPLTFGGGTRVEIK (SEQ ID NO:86) |

| UC antibody 3 |
|---|
| Heavy chain variable region |
| QVQLVESGGGVVQPGRSLRVSCAASGFTFSIYGMHWVRQAPGKGLEWVAVISS DGTNQYYADSVKGRFTISRDNSNNTVYLQMNSLGGEDTAVYYCAKDRGRRGD SGWYRHFDYWGQGTLVTVSS (SEQ ID NO:87) |
| Light chain variable region |
| QSVLTQPPSVSAAPGQKVTISCSGSSSNNGNNYVSWYQQVPGTAPKLLIYDNN KRPSGIPDRFSGSKSGTSATLGITGLQTGDEADYYCGTWDSSLSAVVFGGGTKL TVL (SEQ ID NO:88) |

**[Table 2-2]**

| UC antibody 4 |
|---|
| Heavy chain variable region |
| QVQLVQSGAEVKKPGASVKVSCKASGYTFSSFGISWVRQAPGQGLEWMGWIS AYNGNTNSAQKFQGRVTMTTDTSTSTAYMELRSLRSDDTAVYYCARDRGFRG DTAMIKGGMDVWGQGTTVTVSS (SEQ ID NO:89) |
| Light chain variable region |
| SYELTQPPSVSVSPGQTARITCSGDALPKQYAYWYQQKPGQAPVLVIYKDSERP SGIPERFSGSSSGTMATLTISGAQVEDEGDYYCSSTDSNSQRVFGGGTKLTVL (SEQ ID NO:90) |

| UC antibody 5 |
|---|
| Heavy chain variable region |
| QVQLVQSGAEVKKPGASVKVSCKASGHTFSSFGISWVRQAPGQGLEWMGWIS AYNGNTNSAQKFQGRVTMTTDTSTSTAYMELRSLRSDDTAVYYCARDRGFRG DTAMIKGGMDVWGQGTTVTVSS (SEQ ID NO:91) |
| Light chain variable region |
| VLTQPPSVSVSPGQTASITCSGDKLGDKYACWYQQKSGQAPVLVIYQDSKRPS GIPERFSGSNSGNTATLTISGTQAMDEADYYCQAWDSSTALVFGGGTKLTVL (SEQ ID NO:92) |

| UC antibody 6 |
|---|
| Heavy chain variable region |
| QVQLVQSGAEVKKPGASVKVSCKASGHTFSSFGISWVRQAPGQGLEWMGWIS AYNGNTNSAQKFQGRVTMTTDTSTSTAYMELRSLRSDDTAVYYCARDRGFRG DTAMIKGGMDVWGQGTTVTVSS (SEQ ID NO:93) |
| Light chain variable region |
| QSVLTQPPSVSGAPGQRVTISCTGSSSTIGANNDVHWYQQLPGTAPKLLIYGNK NRPSGVSDRFSGSKSGTSASLAITGLQAEDEADYYCQSYDSSLSDLYVFGTGTK VTVL (SEQ ID NO:94) |

**[Table 2-3]**

| UC antibody 7 |
|---|
| Heavy chain variable region |
| VQLVESGGGVVQPGRSLRLSCAASGFTFSSYGMHWVRQAPGKGLEWVAFISYD GINKYYADSVKGRFTISRDNSKDTLYLQMNSLRAEDTAVYYCAKVIPRIRGSGD KAGIKDYYYYGMDVWGQGTTVTVSS (SEQ ID NO:95) |
| Light chain variable region |
| EIVLTQSPATLSLSPGERATLSCRASQSVSSYLAWYQQKPGQAPRLLIYDASNRA TGIPARFSGSGSGTDFTLTISSLEPEDFAVYYCQQRSNWLTFGGGTKVEIK (SEQ ID NO:96) |

| UC antibody 8 |
|---|
| Heavy chain variable region |
| QVQLVESGGGVVQPGRSLRLSCAASGFTFSSYAMHWVRQAPGKGLEWVAFISY DGINKYYADSVKGRFTISRDNPKDTLYLQMNSLRAEDTAVYYCAKVIPRIRGSG DKAGIKDYYYYGMDVWGRGTTVTVSS (SEQ ID NO:97) |
| Light chain variable region |
| EIVLTQSPGTLSLSPGERATLSCRASQSVSSSYLAWYQQKPGQAPRLLIYGASSR ATGIPDRFSGSGSGTDFTLTISRLEPEDFAVYYCQQYGSSVWTFGQGTKVEIK (SEQ ID NO:98) |

| UC antibody 9 |
|---|
| Heavy chain variable region |
| QVQLVESGGGVVQPGRSLRLSCAASGFTFSSYGMHWVRQAPGKGLEWVAFISY DGINKYYADSVKGRFTISRDNSKDTLYLQMNSLRAEDTAVYYCAKVIPRIRGSG DKAGIKDYYYYGMDVWGQGTTVTVSS (SEQ ID NO:99) |
| Light chain variable region |
| QSVLTQPPSVSAAPGQKVTISCSGSSSNIGNNYVSWYQQLPGTAPKLLIYDNNK RPSGIPDRFSGSKSGTSATLGITGLQTGDEADYYCGTWDSSLSAVVFGGGTKLT VL (SEQ ID NO:100) |

**[Table 2-4]**

| UC antibody 10 |
|---|
| Heavy chain variable region |
| QVQLVQSGAEVKKPGASVKVSCKVSGYTLTELRIHWVRQVPGKGLEWMGGFD PEDGETIYTQKFQGRVTMTEDTSTDTAYMELSSLRSEDTAVYYCTTDLFAFVRG VRGAFDIWGQGTMVTVSS (SEQ ID NO:101) |
| Light chain variable region |
| DIQMTQSPSSLSASVGDRVTITCRASQTITTYLNWYQQKPGKAPKLLIYRASSL HSGVPSRFSGSGSGTDFTLTISSLQPEDFATYYCQQSYRTLWTFGQGTKVEIN T(SEQ ID NO:102) |

| PSC antibody 1 |
|---|
| Heavy chain variable region |
| QVQLVQSGAEVKKPGASVKVSCKASGHTFSSFGISWVRQAPGQGLEWMGWIS AYNGNTNSAQKFQGRVTMTTDTSTSTAYMELRSLRSDDTAVYYCARDRGFRG DTAMIKGGMDVWGQGTTVTVSS (SEQ ID NO:103) |
| Light chain variable region |
| QSALTQPASVSGSPGQSITISCTGTSSDVGGYNYVSWYQQHPGKAPQLIIYDVS KRPSGVSNRFSGSRSGNTASLTISGLQAEDEADYYCSSYTSSSTYVFGTGTKVT VL (SEQ ID NO:104) |

| PSC antibody 2 |
|---|
| Heavy chain variable region |
| EAHLLESGGGLVQPGGSLRLSCEGSGFDFSNYVMNWVRQAPGKGLEWVSAITD RGDSRYYINSVKGRFTISRDNSKNTLFLEMYGLTPEDTAVYYCAKDQTFAGDDP TVFDSWGLGTLVTVSS (SEQ ID NO:105) |
| Light chain variable region |
| QSALTQPASVSGSPGQSITISCTGTSSDIGGYNYVSWYQQHPGKAPKLMIYDV NKRPSGVSNRFSGSKSGNTASLTISGLQAEDEADYYCNSYTTSSTSVFGTGTKV TVL (SEQ ID NO:106) |

### UC Antibody 1:

Heavy chain variable region 
CAGGTGCAGCTGGTGGAGTCTGGGGGAGGCGTGGTCCAGCCTGGGAGGTCCCTGAGACT
CTCCTGTGCAGCCTCTGGATTCACCTTCAGTAGCTATGGCATGCACTGGGTCCGCCAGG
CTCCAGGCAAGGGGCTGGAGTGGGTGGCATTTATATCATATGATGGAATTAATAAATAC
 
TATGCAGACTCCGTGAAGGGCCGCTTC

ACCATCTCCAGAGACAATTCCAAGGACACGCTGTATCTGCAAATGAACAGCCTGAGAGC

TGAGGACACGGCTGTATATTACTGTGCGAAAGTCATCCCCAGGATAAGGGGTTCGGGAG

ACAAAGCGGGGATAAAAGACTACTACTACTACGGTATGGACGTCTGGGGCCAAGGGACC

ACGGTCACCGTCTCCTCA (SEQ ID NO: 107)
Light chain variable region

GATATTGTGATGACTCAGTCTCCACTCTCCCTGCCCGTCACCCCTGGAGAGCCGGCCTC
CATCTCCTGCAGGTCTAGTCAGAACCTCCTGCATAGTAATGGATACAACTATTTGGATT
GGTACCTGCAGAAGCCAGGGCAGTCTCCACAGCTCCTGATCTATTTGGGTTCT
AATCGGGCCTCCGGGGTCCCTGACAGGTTCAGTGGCAGTGGATCAGGCACAGATTTTAC
ACTGAAAATCAGCAGAGTGGAGGCTGAGGATGTTGGGGTTTATTACTGCATGCAAGCTC
TACAAACTTGGACGTTCGGCCAAGGGACCAAGGTGGAAATCAAA (SEQ ID NO:
108)

### UC Antibody 2:

Heavy chain variable region 
GAGGTGCAGTTGGTGGAGTCTGGGGGAGGCTTGGTAAAGCCTGGGGGGTCCCTTAGAGT
CTCCTGTGTAGTCTCTGGATTCACAATCACTAACGCCTGGATGAGCTGGGTCCGCCAGG
CTCCAGGGAAGGGGCTGGAGTGGGTTGGCCGTAGTAAAAGCAAAACTGATGGTGGGACA
ACAGACTACATTGCACCCGTGAAAGGCAGATTCACCATCTCAAGAGATGATTCAAAAAA
CACACTTTATCTGGAAATGAACAGCCTGAAAACCGAGGACACAGCCGTGTATTACTGTG
CCACAGATCGGCCTCTGAAACTAAGGGGTAGAGACTACAACTACTACGTTATGGACGTC
TGGGGCCAAGGGACCACGGTCACCGTCTCCTCA (SEQ ID NO: 109)
Light chain variable region

GACATCCAGATGACCCAGTCTCCATCCTCCCTGTCTGCTTCTGTTGGAGACAGAGTCAC
CATCACTTGCCGGGCAAGTCAGAGCATTGCCAGATCTTTAAATTGGTATCAGCAAAAAC
CAGGGAAAGCCCCTAATCTCCTGATCTATGCTGCATCCACTTTGCAA
AGTGGGGTCCCATCAAGGTTCAGTGGCAGTGGATCTGGGACAGATTTCACTCTCACCAT
CGGCAGTCTGCAACCTGAAGATTTCGCAACTTACTCCTGTCAACAGAGTTCCAGTTCCC
CTCTCACTTTCGGCGGAGGGACCAGGGTGGAGATCAAG (SEQ ID NO: 110)

### UC antibody 3:

Heavy chain variable region 
CAGGTGCAGCTGGTGGAGTCTGGGGGAGGCGTGGTCCAGCCTGGGAGGTCCCTGAGAGT
CTCCTGTGCAGCCTCTGGATTCACCTTCAGTATTTATGGGATGCACTGGGTCCGCCAGGC
TCCAGGCAAGGGGCTGGAGTGGGTGGCAGTTATTTCATCTGATGGAACTAATCAATACT
ATGCAGACTCCGTG
AAGGGCCGATTCACCATCTCCAGAGACAATTCCAACAACACGGTGTATCTGCAAATGAA
CAGCCTGGGAGGTGAGGACACGGCTGTGTATTACTGTGCGAAAGATCGGGGCCGCCGGG
GGGACAGTGGCTGGTACCGACACTTTGACTACTGGGGCCAGGGAACCCTGGTCACCGTCT
CCTCA (SEQ ID NO: 111)
Light chain variable region

CAGTCTGTGTTGACGCAGCCGCCCTCAGTGTCTGCGGCCCCAGGACAGAAGGTCACCATC
TCCTGCTCTGGAAGCAGCTCCAACAATGGGAATAATTATGTATCCTGGTACCAGCAGGT
CCCAGGAACAGCCCCCAAACTCCTCATTTATGACAATAATAAGCGACCCT
CAGGGATTCCTGACCGATTCTCTGGCTCCAAGTCTGGCACGTCAGCCACCCTGGGCATC
ACCGGACTCCAGACTGGGGACGAGGCCGATTATTACTGCGGAACATGGGATAGCAGCCT
GAGTGCTGTAGTCTTCGGCGGAGGGACCAAGCTGACCGTCCTA (SEQ ID NO: 112)

### UC Antibody 4:

Heavy chain variable region 
CAGGTTCAGCTGGTGCAGTCTGGAGCTGAGGTGAAGAAGCCTGGGGCCTCAGTGAAGGT
CTCCTGCAAGGCTTCTGGTTACACCTTTTCCAGCTTTGGTATCAGCTGGGTGCGACAGG
CCCCTGGACAAGGGCTTGAGTGGATGGGATGGATCAGCGCTTACAATGGTAACACAAAC
TCTGCACAGAAGTTCCAGGGCAGAGTCACCATGACCACAGACACATCCACGAGCACAGC
CTACATGGAGCTGAGGAGCCTGAGATCTGACGACACGGCCGTGTATTACTGTGCGAGAG
ATAGGGGTTTTCGCGGGGACACAGCTATGATTAAAGGGGGTATGGACGTCTGGGGCCAAG
GGACCACGGTCACCGTCTCCTCA (SEQ ID NO: 113)
Light chain variable region

TCCTATGAGCTGACACAGCCACCCTCGGTGTCAGTGTCCCCAGGACAGACGGCCAGGAT

CACCTGCTCTGGAGATGCATTGCCAAAGCAATATGCTTATTGGTACCAGCAGAAGCCAG
GCCAGGCCCCTGTGCTGGTGATATATAAAGACAGTGAGAGGCCCTCA
GGGATCCCTGAGCGATTCTCTGGCTCCAGCTCAGGGACAATGGCCACCTTGACTATCAG
TGGGGCCCAGGTGGAGGATGAAGGTGACTACTACTGTTCCTCAACAGACAGCAATTCTC
AGCGCGTATTCGGCGGAGGGACCAAGCTGACCGTCCTA (SEQ ID NO: 114)

### UC Antibody 5:

Heavy chain variable region 
CAGGTTCAGCTGGTGCAGTCTGGAGCTGAGGTGAAGAAGCCTGGGGCCTCAGTGAAGGT
CTCCTGCAAGGCTTCTGGTCACACCTTTTCCAGCTTTGGTATCAGCTGGGTGCGACAGG
CCCCTGGACAAGGGCTTGAGTGGATGGGATGGATCAGCGCTTACAATGGTAACACAAAC
TCTGCACAGAAGTTCCAGGGCAGAGTCACCATGACCACAGACACATCCACGAGCACAGC
CTACATGGAGCTGAGGAGCCTGAGATCTGACGACACGGCCGTGTATTACTGTGCGAGAG
ATAGGGGTTTTCGCGGGGACACAGCTATGATTAAAGGGGGTATGGACGTCTGGGGCCA
AGGGACCACGGTCACCGTCTCCTCA (SEQ ID NO: 115)
Light chain variable region

GTGCTGACGCAGCCGCCCTCAGTGTCCGTGTCCCCAGGACAGACAGCCAGCATCACCTGC
TCTGGAGATAAATTGGGGGATAAATATGCTTGCTGGTACCAGCAGAAGTCAGGCCAGGC
CCCTGTATTGGTCATCTATCAAGATAGCAAGCGGCCCTCAGGG
ATCCCTGAGCGATTCTCTGGCTCCAACTCTGGGAACACAGCCACTCTGACCATCAGCGG
GACCCAGGCTATGGATGAGGCTGACTATTACTGTCAGGCGTGGGACAGCAGCACTGCGC
TGGTATTCGGCGGAGGGACCAAGCTGACCGTCCTA (SEQ ID NO: 116)

### UC Antibody 6:

Heavy chain variable region 
CAGGTTCAGCTGGTGCAGTCTGGAGCTGAGGTGAAGAAGCCTGGGGCCTCAGTGAAGGT
CTCCTGCAAGGCTTCTGGTCACACCTTTTCCAGCTTTGGTATCAGCTGGGTGCGACAGG
CCCCTGGACAAGGGCTTGAGTGGATGGGATGGATCAGCGCTTACAATGGTAACACAAAC
TCTGCACAGAAGTTCCAGGGCAGAGTCACCATGACCACAGACACATCCACGAGCACAGC
CTACATGGAGCTGAGGAGCCTGAGATCTGACGACACGGCCGTGTATTACTGTGCGAGAG
 
ATAGGGGTTTTCGCGGGGACACAGCTATGATTAAAGGGGGTATGGACGTCTGGGGCCAAG
GGACCACGGTCACCGTCTCCTCA (SEQ ID NO: 117)
Light chain variable region

CAGTCTGTGCTGACGCAGCCGCCCTCAGTGTCTGGGGCCCCAGGGCAGAGGGTCACCAT
CTCCTGCACTGGGAGCAGTTCCACCATCGGGGCAAATAATGATGTACACTGGTACCAGCA
ACTTCCAGGAACAGCCCCCAAACTCCTCATCTATGGTAACAAGAATCGCCCCT
CAGGGGTCTCTGACCGATTCTCTGGCTCCAAGTCTGGCACCTCAGCCTCCCTGGCCATC
ACTGGGCTCCAGGCTGAGGATGAGGCTGATTATTACTGCCAGTCCTATGACAGCAGTCT
GAGTGATCTTTATGTCTTCGGAACGGGGACCAAGGTCACCGTCCTA (SEQ ID NO:
118)

### UC Antibody 7:

Heavy chain variable region 
GTGCAGCTGGTGGAGTCTGGAGGAGGCGTGGTCCAGCCTGGGAGGTCCCTGAGACTCTC
CTGTGCAGCCTCTGGATTCACCTTCAGTAGCTATGGCATGCACTGGGTCCGCCAGGCTC
CAGGCAAGGGGCTGGAGTGGGTGGCATTTATATCATATGATGGAATTAATAAATACTAT
GCAGACTCCGTGAAGGGCCGCTTCA
CCATCTCCAGAGACAATTCCAAGGACACGCTGTATCTGCAAATGAACAGCCTGAGAGCT
GAGGACACGGCTGTATATTACTGTGCGAAAGTCATCCCCAGGATAAGGGGTTCGGGAGA
CAAAGCGGGGATAAAAGACTACTACTACTACGGTATGGACGTCTGGGGCCAAGGGACCA
CGGTCACCGTCTCCTCA (SEQ ID NO: 119)
Light chain variable region

GAAATTGTGTTGACACAGTCTCCAGCCACCCTGTCTTTGTCTCCAGGGGAAAGAGCCAC
CCTCTCCTGCAGGGCCAGTCAGAGTGTTAGCAGCTACTTAGCCTGGTACCAACAGAAAC
CTGGCCAGGCTCCCAGGCTCCTCATCTATGATGCATCCAACAGGG
CCACTGGCATCCCAGCCAGGTTCAGTGGCAGTGGGTCTGGGACAGACTTCACTCTCACC
ATCAGCAGCCTAGAGCCTGAAGATTTTGCAGTTTATTACTGTCAGCAGCGTAGCAACTG
GCTCACTTTCGGCGGAGGGACCAAGGTGGAGATCAAA (SEQ ID NO: 120)

### UC Antibody 8:

Heavy chain variable region 
CAGGTGCAGCTGGTGGAGTCTGGGGGAGGCGTGGTCCAACCTGGGAGGTCCCTGAGACT
CTCCTGTGCAGCCTCTGGATTCACCTTCAGTAGCTATGCTATGCACTGGGTCCGCCAGG
CTCCAGGCAAGGGGCTGGAGTGGGTGGCATTTATATCATATGATGGAATTAATAAATAC
TATGCAGACTCCGTGAAGGGCCGCTTC
ACCATCTCCAGAGACAATCCCAAGGACACGCTGTATCTGCAAATGAACAGCCTGAGAGC
TGAGGACACGGCTGTATATTACTGTGCGAAAGTCATCCCCAGGATAAGGGGTTCGGGAG
ACAAAGCGGGGATAAAAGACTACTACTACGGTATGGACGTCTGGGGCCGAGGGACCACG
GTCACCGTCTCCTCA (SEQ ID NO: 121)
Light chain variable region

GAAATTGTGTTGACACAGTCTCCAGGCACCCTGTCTTTGTCTCCAGGGGAAAGAGCCAC
CCTCTCCTGCAGGGCCAGTCAGAGTGTTAGCAGCAGCTACTTAGCCTGGTACCAGCAGA
AACCTGGCCAGGCTCCCAGGCTCCTCATCTATGGTGCATCCAGCAGGG
CCACTGGCATCCCAGACAGGTTCAGTGGCAGTGGGTCTGGGACAGACTTCACTCTCACC
ATCAGCAGACTGGAGCCTGAAGATTTTGCAGTGTATTACTGTCAGCAGTATGGTAGCTC
AGTGTGGACGTTCGGCCAAGGGACCAAGGTGGAAATCAAA (SEQ ID NO: 122)

### UC Antibody 9:

Heavy chain variable region 
CAGGTGCAGCTGGTGGAGTCTGGGGGAGGCGTGGTCCAGCCTGGGAGGTCCCTGAGACT
CTCCTGTGCAGCCTCTGGATTCACCTTCAGTAGCTATGGCATGCACTGGGTCCGCCAGG
CTCCAGGCAAGGGGCTGGAGTGGGTGGCATTTATATCATATGATGGAATTAATAAATAC
TATGCAGACTCCGTGAAGGGCCGCTTC
ACCATCTCCAGAGACAATTCCAAGGACACGCTGTATCTGCAAATGAACAGCCTGAGAGC
TGAGGACACGGCTGTATATTACTGTGCGAAAGTCATCCCCAGGATAAGGGGTTCGGGAG
ACAAAGCGGGGATAAAAGACTACTACTACTACGGTATGGACGTCTGGGGCCAAGGGACC
ACGGTCACCGTCTCCTCA (SEQ ID NO: 123)
Light chain variable region

CAGTCTGTGTTGACGCAGCCGCCCTCAGTGTCTGCGGCCCCAGGACAGAAGGTCACCAT
CTCCTGCTCTGGAAGCAGCTCCAACATTGGGAATAATTATGTATCCTGGTACCAGCAGC

TCCCAGGAACAGCCCCCAAACTCCTCATTTATGACAATAATAAGCGACCCT

CAGGGATTCCTGACCGATTCTCTGGCTCCAAGTCTGGCACGTCAGCCACCCTGGGCATC

ACCGGACTCCAGACTGGGGACGAGGCCGATTATTACTGCGGAACATGGGATAGCAGCCT

GAGTGCTGTGGTATTCGGCGGAGGGACCAAGCTGACCGTCCTA (SEQ ID NO: 124)

### UC Antibody 10:

Heavy chain variable region 
CAGGTCCAGCTGGTACAGTCTGGGGCTGAGGTGAAGAAGCCTGGGGCCTCAGTGAAAGT
CTCCTGCAAGGTTTCCGGATACACCCTCACTGAATTACGCATACACTGGGTGCGACAGG
TTCCTGGAAAAGGGCTTGAGTGGATGGGAGGTTTTGATCCTGAAGATGGTGAAACAATC
TACACACAGAAGTTCCAGGGCAGAGTCACGATGACCGAGGACACATCTACAGACACAGC
CTACATGGAGCTGAGCAGCCTGAGATCTGAGGACACGGCCGTCTATTACTGTACAACAG
ATCTTTTCGCTTTCGTTCGGGGAGTTAGGGGTGCTTTTGATATCTGGGGCCAGGGGACA
ATGGTCACCGTCTCTTCA (SEQ ID NO: 125)
Light chain variable region

GACATCCAGATGACCCAGTCTCCATCCTCCCTGTCTGCATCTGTAGGAGACAGAGTCAC
CATCACTTGCCGGGCAAGTCAGACCATTACTACTTATTTAAATTGGTATCAGCAGAAAC
CAGGGAAAGCCCCTAAGCTCCTGATCTATAGAGCATCCAGTTTGCAC
AGTGGGGTCCCATCTAGGTTCAGTGGCAGTGGATCTGGGACAGATTTCACTCTCACCAT
CAGCAGTCTGCAACCTGAAGATTTTGCAACTTACTACTGTCAACAGAGTTACCGTACCC
TCTGGACGTTCGGCCAAGGGACCAAGGTGGAAATCAAC (SEQ ID NO: 126)

### PSC Antibody 1:

Heavy chain variable region 
CAGGTTCAGCTGGTGCAGTCTGGAGCTGAGGTGAAGAAGCCTGGGGCCTCAGTGAAGGT
CTCCTGCAAGGCTTCTGGTCACACCTTTTCCAGCTTTGGTATCAGCTGGGTGCGACAGG
CCCCTGGACAAGGGCTTGAGTGGATGGGATGGATCAGCGCTTACAATGGTAACACAAAC
TCTGCACAGAAGTTCCAGGGCAGAGTCACCATGACCACAGACACATCCACGAGCACAGC
CTACATGGAGCTGAGGAGCCTGAGATCTGACGACACGGCCGTGTATTACTGTGCGAGAG
ATAGGGGTTTTCGCGGGGACACAGCTATGATTAAAGGGGGTATGGACGTCTGGGGCCA
 AGGGACCACGGTCACCGTCTCCTCA (SEQ ID NO: 127)
Light chain variable region

CAGTCTGCCCTGACTCAGCCTGCCTCCGTGTCTGGGTCTCCTGGACAGTCGATCACCAT
CTCCTGCACTGGAACCAGCAGTGACGTTGGTGGTTATAACTATGTCTCCTGGTACCAA
CAGCACCCAGGCAAAGCCCCCCAACTCATAATTTATGATGTCAGTAAGCGGC
CCTCAGGGGTTTCTAATCGCTTCTCTGGCTCCAGGTCTGGCAACACGGCCTCCCTGACC
ATCTCTGGGCTCCAGGCTGAGGACGAGGCTGATTATTACTGCAGCTCATATACAAGCAG
CAGCACTTATGTCTTCGGAACTGGGACCAAGGTCACCGTCCTA (SEQ ID NO: 128)

### PSC Antibody 2:

Heavy chain variable region 
GAGGCGCATCTGTTGGAGTCTGGGGGAGGCCTGGTACAGCCTGGGGGGTCCCTGAGACT
CTCATGTGAAGGCTCTGGGTTCGACTTTAGCAATTATGTCATGAACTGGGTCCGCCAGGC
TCCAGGGAAGGGGCTGGAGTGGGTCTCCGCAATTACTGACAGAGGTGATAGTCGATACT
ATATAAATTCAGTGAAGGGCCGGTTCACCATCTCCAGAGACAATTCCAAGAACACGCTG
TTTCTGGAGATGTACGGCCTGACACCCGAAGACACGGCCGTCTATTACTGTGCCAAGGA
TCAGACATTTGCGGGCGACGACCCCACCGTCTTCGACTCCTGGGGCCTGGGAACCCTGG
TCACCGTCTCCTCCA (SEQ ID NO: 129)
Light chain variable region

CAGTCTGCCCTGACTCAGCCTGCCTCCGTGTCTGGGTCTCCTGGACAGTCGATCACCAT
CTCCTGCACTGGAACCAGCAGTGACATTGGTGGTTATAACTATGTCTCCTGGTACCAAC
AACACCCAGGCAAAGCCCCCAAACTCATGATTTATGATGTCAATAAGCGGC
CCTCAGGGGTTTCTAATCGCTTCTCTGGCTCCAAGTCTGGCAACACGGCCTCCCTGACCA
TCTCTGGGCTCCAGGCTGAGGACGAGGCTGATTATTACTGCAACTCATATACAACCAGC
AGCACTTCTGTCTTCGGAACTGGGACCAAGGTCACCGTCCTA (SEQ ID NO: 130)

### 2. Inhibition test of binding to integrin αvβ6

Furthermore, a competitive assay with fibronectin for binding to integrin αvβ6 was performed to confirm the function of the obtained monoclonal antibodies. Briefly, a 96-well microtiter plate was coated with 150 µL/well of 2 µg/mL human integrin αvβ6 (ACROBiosystems, Cat. No. IT6-H52E1) overnight at 4°C, then blocked and incubated with 120 µL of monoclonal antibodies (0.0001-10 µg/mL) for 60 min at room temperature. After washing, the plate was incubated with 100 µL of 2 µg/mL fibronectin (FC010; MilliporeSigma, Burlington, MA) for 60 minutes at room temperature. After washing, an anti-fibronectin antibody (1:5000 dilution; ab2413; Abcam) was added to the plate, and the plate was incubated for 60 min at room temperature. After washing, an HRP-labeled anti-rabbit IgG antibody (1:10,000 dilution; A27036; ThermoFisher Scientific) was added to the plate, and the plate was incubated at room temperature for 60 min. After washing, the plate was incubated with 3,3',5,5'-tetramethylbenzidine for 10 minutes, and the absorbance at 450 nm was measured to detect fibronectin bound to integrin αvβ6 on the solid phase. The assay was carried out in the presence of MgCl₂ (1 mM) and CaCl₂ (1 mM). To calculate the binding inhibition rate, blank wells coated with integrin αvβ6 were incubated with fibronectin in the absence of antibody, and the optical density (OD) at 450 nm was measured in the same manner as above. The binding inhibition rate (%) was calculated according to the following formula. Results are shown in Figure 6. Binding inhibition rate (%) = {[(OD value of blank well) - (OD value of well to which antibody was added)]/(OD value of blank well)} × 100

All of the monoclonal antibodies tested (UC antibodies 1 to 8, PSC antibody 1) inhibited the binding of fibronectin to integrin αvβ6 (Figure 6).

CDR3 is the most important site for antibody specificity (binding ability). Thus, it was found that an epitope for the anti-integrin αvβ6 autoantibody derived from patients with ulcerative colitis and primary sclerosing cholangitis is a portion containing the RGD binding site on integrin αvβ6. It was also found that in ulcerative colitis or primary sclerosing cholangitis, the anti-integrin αvβ6 autoantibody competes with RGD of fibronectin, and thereby the binding between fibronectin and integrin αvβ6 is inhibited.

### 3. Binding affinity analysis

Next, the binding affinity of the obtained anti-integrin αvβ6 monoclonal antibodies derived from UC or PSC patients was determined. The KD value was measured by biolayer interferometry using Octet RED96 (Sartorius). A 100 nM solution of the antibody, and serially diluted solutions of biotinylated integrin αvβ6 protein in the range of 200 to 6.25 nM were prepared using a buffer containing 20 mM Tris, 150 mM NaCl, 1 mM CaCl₂, 1 mM MgCl₂, 0.1% human serum albumin (014-27604; Fujifilm Wako Pure Chemical Industries, Ltd.) and 0.02% Tween 20 (1610781; Bio-Rad), and added to a 96-well plate (Greiner; 655209) at 200 µL/well. The biotinylated integrin αvβ6 was allowed to bind for 5 minutes to a biosensor (18-5136; Sartorius) soaked in the buffer. After washing of the biosensor, the biosensor was immersed in each antibody solution for 5 minutes (binding reaction). Then, the sensor was immersed in the buffer solution for 1 minute (dissociation reaction). The binding dissociation constant (KD value) between "integrin αvβ6-each monoclonal antibody" was determined using the analysis software (Octet BLI Analysis) attached to the device. Results are shown in Table 3.

**[Table 3]**

| monoclonal antibody | KD value (nM) |
|---|---|
| UC antibody 1 | 4.54 |
| UC antibody 2 | 1.95 |
| UC antibody 3 | 15.04 |
| UC antibody 4 | 3.15 |
| UC antibody 5 | 8.25 |
| UC antibody 6 | 20.80 |
| UC antibody 7 | 5.18 |
| UC antibody 8 | 10.63 |
| UC antibody 9 | 5.96 |
| UC antibody 10 | NA |
| PSC antibody 1 | 8.91 |
| PSC antibody 2 | NA |

The KD values of UC antibodies 1, 2, 4, 5, 7, 9 and PSC antibody 1 were on the order of 1x10⁻⁹ M, and the KD values of UC antibodies 3, 6 and 8 were on the order of 1x10⁻⁸ M. Thus it was found that these antibodies have high binding affinity for integrin αvβ6. UC antibodies 3, 6 and 8 were consistent with the three antibodies that had the least inhibitory effect on fibronectin binding to integrin αvβ6 (Figure 6). The KD values of UC antibody 10 and PSC antibody 2 were not measurable, suggesting that these antibodies have low binding affinity to integrin αvβ6.

### <Example 3. Preparation of primary sclerosing cholangitis animal model>

Eight-week-old mice (ten C57BL/6J mice, and five BALB/c mice) were immunized by subcutaneous injection of 100 µg/mouse of human integrin αvβ6 (ACROBiosystems, product number IT6-H52E1) together with an adjuvant [CFA (Freund's Complete Adjuvant, manufacturer; BD, product number: 263810) or IFA (Freund's Incomplete Adjuvant, manufacturer; BD, product number: 263910)] into the back (day 0), and then immunized with the same amount of antigen as the first time on days 14 and 28 to induce production of anti-integrin αvβ6 antibodies. As a control, mice were similarly immunized with OVA (ovalbumin).

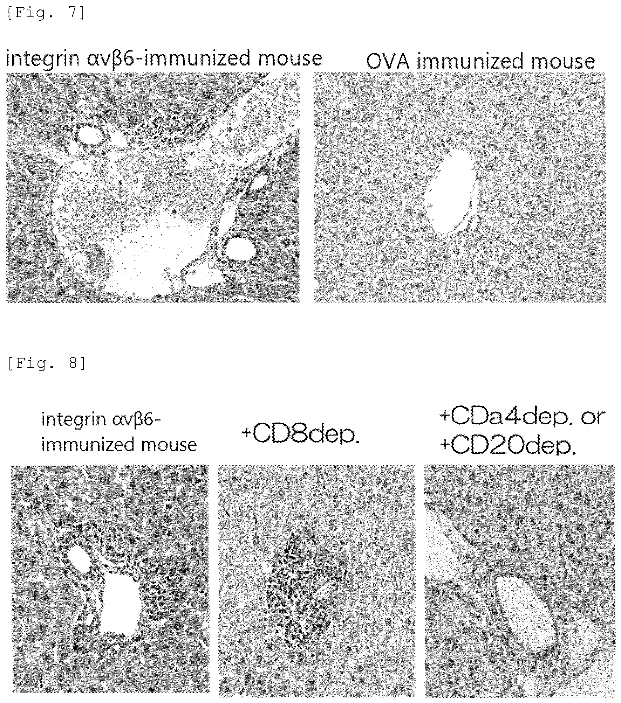

The mice were sacrificed 56 days after immunization, and the bile ducts of the mice were observed. Partial loss of the bile ducts and cellular infiltration around the bile ducts were observed in the integrin αvβ6-immunized mice (Figure 7). These are similar to the pathological findings of PSC. All integrin αvβ6-immunized mice had cellular infiltration in the bile ducts consistent with PSC pathology. On the other hand, in the control OVA-immunized mice, neither cellular infiltration around the bile ducts nor loss of the bile ducts was observed.

### <Example 4. Examination of mechanism in primary sclerosing cholangitis animal model>

In order to determine which element, cellular immunity (immunity mainly through T cells) or humoral immunity (immunity mainly through B cells that fights against foreign substances by producing antibodies), had more highly affected the PSC model mice prepared in Example 3, anti-CD8 antibody (BioXcell, InVivoMab anti-mouse CD8a Clone: 2.43), anti-CD20 antibody (BioXcell, InVivoMAb anti-mouse CD20 Clone: AISB12), or anti-CD4 antibody (BioXcell, InVivoMab anti-mouse CD4 Clone: GK1.5) as a depleting antibody was administered to the mice. the anti-CD8 antibody suppresses cellular immunity, the anti-CD20 antibody suppresses humoral immunity, and the anti-CD4 antibody suppresses both cellular immunity and humoral immunity.

As a result, suppression of cell infiltration was observed when the anti-CD20 antibody or the anti-CD4 antibody was administered ("+CDa4dep. or +CD20dep." in Figure 8). On the other hand, when the anti-CD8 antibody was administered, there was no change in the pathological condition ("+CD8dep." in Figure 8). Therefore, it was thought that antibody production by humoral immunity (herein, production of anti-integrin αvβ6 antibodies) causes the pathology of PSC.

### <Example 5. Creation of integrin αvβ6 knockout animal>

In C57BL/6J mice, Exon 3 and Exon 4 of the integrin β6 gene were deleted and gene editing was performed so as to insert a stop codon into Exon 5 to create integrin αvβ6 gene knockout mice (n=5), using CRISPR/Cas9 gene editing technology. There are several types of β that bind to αv, whereas only αv binds to β6. Thus, knocking out the integrin αvβ6 gene results in knocking out the integrin αvβ6 protein (making only the integrin αvβ6 protein functionally defective, among the integrin family proteins).

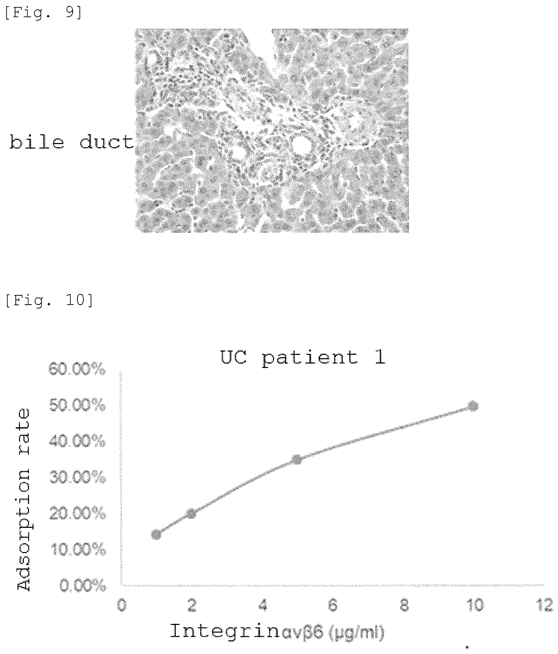

Figure 9 shows a photomicrograph of the bile duct in the mouse lacking the function of integrin αvβ6 protein. In integrin β6 gene knockout mice (mice lacking the function of integrin αvβ6 protein), partial loss of the bile ducts and inflammatory cell infiltration (lymphocyte infiltration) around the bile ducts were observed (Figure 9). Therefore, the finding in Example 4 that inhibition of integrin αvβ6 function by the anti-integrin αvβ6 antibody is involved in the pathogenesis of PSC was supported.

### <Example 6. Antibody removal experiment using anti-integrin αvβ6 antibody adsorption column -1>

Using a commercially available histidine tag purification kit (His tagged Protein Purification Kit, MBL, #3310), histidine-tagged extracellular domain protein of integrin αvβ6 (10 µg/mL; ACROBiosystems, #IT6-H52E1) and serum samples (50 µL) obtained from ulcerative colitis patients (n=3) were placed in a column packed with anti-histidine tag beads (agarose beads bound to anti-histidine tag antibodies) and allowed to react. Then, the sample that fell off the column (the sample that passed through the column) was collected by centrifugation. A microtiter plate was coated with 100 µL/well of 2 µg/mL integrin αvβ6 overnight at 4°C, followed by washing and blocking. After washing, the column-passed sample was diluted 10-fold and added at 100 µL/well to the plate, and the plate was incubated at room temperature for 60 minutes. After washing, an HRP-labeled anti-human IgG antibody (50,000-fold dilution; ab6759; Abcam) was added at 100 µL/well to the plate, and the plate was incubated at room temperature for 60 minutes. After washing, the plate was incubated with 3,3',5,5'-tetramethylbenzidine for 7 minutes, and the absorbance density (OD value) at 450 nm was measured to detect human IgG bound to integrin αvβ6 on the solid phase.

Furthermore, for concentration-dependent experiments, experiments were performed in the same manner as described above, using 1, 2, 5, or 10 µg/mL of histidine-tagged integrin αvβ6 and 25 µL of a serum sample from an ulcerative colitis patient. However, in measuring the absorbance density (OD value) at 450 nm, the column-passed sample was diluted 5-fold before use.

As a control, only the patient's serum was added to the column, and the absorbance density (OD value) at 450 nm of the serum that passed through the column was measured in the same manner as described above. The column adsorption rate of the anti-integrin αvβ6 antibody was calculated according to the following formula: Adsorption rate (%) = [{(OD value of control) - (OD value of sample)}/(OD value of control)] × 100 wherein (as well as in Tables 2 and 3 described below), "sample" refers to the sample that passed through the column when histidine-tagged integrin αvβ6 and a patient serum sample were added to the column.

Results are shown in Tables 4 and 5 and Figure 10. As expected, use of integrin αvβ6 resulted in adsorption of anti-integrin αvβ6 antibodies in the patient serum onto the column. Moreover, the antibody adsorption reaction was dependent on the concentration of integrin αvβ6 to be reacted (Figure 10).

**[Table 4]**

| patient | addition amount of serum (µL) | addition amount of integrin αvβ6 (µg/mL) | sample (A450) | control (A450) | adsorption rate |
|---|---|---|---|---|---|
| UC patient 1 | 50 | 10 | 0.9260 | 1.3784 | 32.82% |
| UC patient 2 | 50 | 10 | 0.1010 | 1.8120 | 94.43% |
| UC patient 3 | 50 | 10 | 0.4986 | 0.8931 | 44.17% |

**[Table 5]**

| patient | addition amount of serum (µL) | addition amount of integrin αvβ6 (µg/mL) | sample (A450) | control (A450) | adsorption rate |
|---|---|---|---|---|---|
| UC patient 1 | 25 | 1 | 1.3853 | 1.6173 | 14.34% |
| UC patient 1 | 25 | 2 | 1.2923 | 1.6173 | 20.10% |
| UC patient 1 | 25 | 5 | 1.0513 | 1.6173 | 35.00% |
| UC patient 1 | 25 | 10 | 0.8123 | 1.6173 | 49.77% |

### <Example 7. Antibody removal experiment using anti-integrin αvβ6 antibody adsorption column -2>

An adsorption test of anti-integrin αvβ6 antibodies contained in sera from patients with ulcerative colitis and primary sclerosing cholangitis was conducted using an adsorbent in which histidine-tagged integrin αvβ6 protein was immobilized on anti-histidine tag antibody-labeled magnetic agarose.

### Antibody adsorption test:

Histidine-tagged integrin αvβ6 (ACRO Biosystems, IT6-H52E1) in the amount of 5 µg/mL or 25 µg/mL was mixed with 10 µL (5 µg of anti-his tag antibody) of anti-histidine tag antibody-labeled magnetic agarose (MBL, D291-10) and 25 µL of the serum from patients with ulcerative colitis (n=8, UC patients 4-11) or primary sclerosing cholangitis (n=9, PSC patients 1-9), and a total of 500 µL of solution was reacted by inversion mixing at 4°C for 12 hours. After the reaction, a tube that had been subjected to the reaction by inversion mixing was placed on a magnetic rack to recover the reaction solution. As a control, a mixture to which histidine-tagged integrin αvβ6 was not added (a mixture of the above-mentioned agarose and patient serum only) was used to perform the similar reaction.

### Measurement of anti-integrin αvβ6 antibody concentration:

The concentration of anti-integrin αvβ6 antibodies contained in the recovered solution was measured by ELISA. A microtiter plate was coated with 100 µL/well of 2 pg/mL integrin αvβ6 overnight at 4°C, followed by washing and blocking. After washing, the recovered reaction solution was diluted 10-fold and added at 100 µL/well to the plate, and the plate was incubated at room temperature for 60 minutes. After washing, an HRP-labeled anti-human IgG antibody (50,000-fold dilution; ab6759; Abcam) was added at 100 µL/well to the plate, and the plate was incubated at room temperature for 60 minutes. After washing, the plate was incubated with 3,3',5,5'-tetramethylbenzidine for 7 minutes, and the absorbance density (OD value) at 450 nm was measured to detect human IgG bound to integrin αvβ6 on the solid phase.

### Conversion of anti-integrin αvβ6 antibody concentration:

To a microtiter plate comprising 2 µg/mL of solid-phased integrin αvβ6, 1 to 0.001 µg/mL of UC antibody 1 (see Table 1) prepared in Example 2 and diluted in a 3-fold common ratio was added, and incubated at room temperature for 60 minutes. After washing of the plate, 100 µL/well of an HRP-labeled anti-human IgG antibody (50,000-fold dilution; ab6759; Abcam) as a secondary antibody was added to the plate, and the plate was incubated at room temperature for 60 minutes. Then, the plate was incubated with 3,3',5,5'-tetramethylbenzidine for 7 minutes, and the optical density at 450 nm (OD value) was measured. Next, a calibration curve was prepared to determine the concentration of anti-integrin αvβ6 antibodies from the obtained OD values. Using the calibration curve, the concentration of anti-integrin αvβ6 antibodies contained in the solution that had been subjected to the antibody adsorption reaction was determined. The adsorption rate of anti-integrin αvβ6 antibodies in the antibody adsorption reaction was calculated from the following formula. An adsorbed sample refers to a solution recovered after the binding reaction to integrin αvβ6 on the solid phase. Adsorption rate (%) = [{(antibody concentration of control) - (antibody concentration of adsorbed sample)}/(antibody concentration of control)] x 100

### Results:

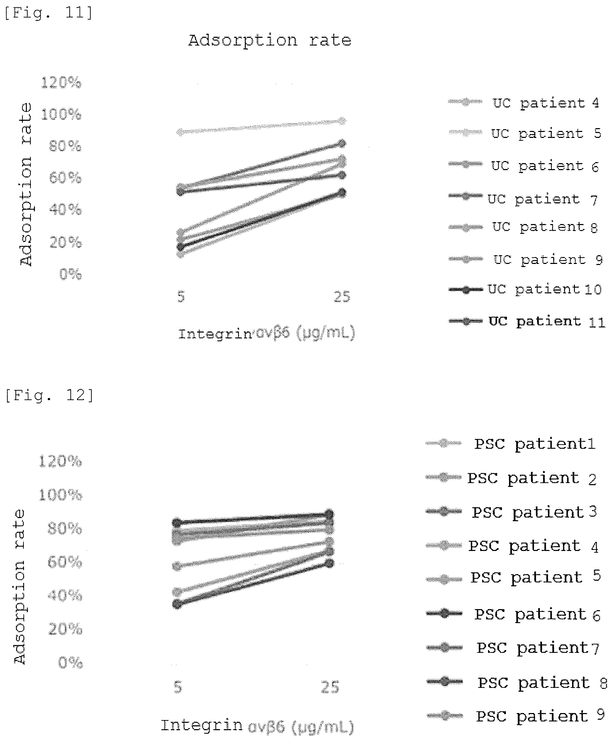

Results are shown in Figures 11 and 12. As is clear from Figures 11 and 12, the adsorbed amounts of anti-integrin αvβ6 antibodies contained in the sera derived from UC patients and PSC patients increased with an increase in the amount of integrin αvβ6 added to the antibody adsorption reaction. When the addition amount of integrin was 25 µg/mL, the adsorption rates of autoantibodies were 50% or more in all samples derived from UC or PSC patients.

### <Example 8. Specificity test for ligand protein>

There are known five types of integrin β subunits for forming heterodimers with integrin αv: β1, β3, β5, β6, and β8. An adsorption test of a monoclonal autoantibody derived from a patient with ulcerative colitis was performed using an adsorbent in which the five types of histidine-tagged integrin proteins were immobilized on anti-histidine tag antibody-labeled agarose, to determine whether the adsorption of the autoantibody was specific to integrin αvβ6.

Anti-histidine tag antibody-labeled magnetic agarose (MBL, D291-10) in the amount of 10 µL (5 µg of anti-his tag antibody), 150 ng of the monoclonal autoantibody derived from an ulcerative colitis patient prepared in Example 2 (UC antibody 1 shown in Table 1), and 5 µg/mL or 25 pg/mL of any of integrins as mentioned below were mixed and reacted by inversion mixing in a total of 500 µL of solution at 4°C for 12 hours. The solution after the reaction was recovered. As a control, a mixture to which no integrin was added (a mixture of the above-mentioned agarose and autoantibody only) was used to perform the similar reaction.
Integrin αvβ1 (ACRO Biosystems, IT1-H82W6)
Integrin αvβ3 (ACRO Biosystems, IT3-H52E3)
Integrin αvβ5 (ACRO Biosystems, IT5-H52W5)
Integrin αvβ6 (ACRO Biosystems, IT6-H52E1)
Integrin αvβ8 (ACRO Biosystems, IT8-H52W4)

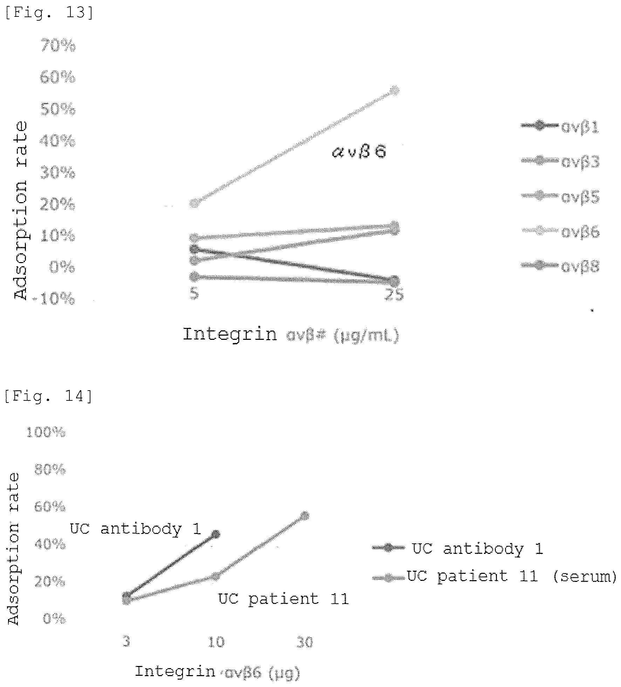

In the same manner as described in Example 7, the concentration of anti-integrin αvβ6 antibody contained in the recovered solution was determined, and the adsorption rate of anti-integrin αvβ6 antibody in the antibody adsorption reaction was calculated. Results are shown in Figure 13.

As is clear from Figure 13, the anti-integrin αvβ6 antibody was adsorbed only when integrin αvβ6 was used as a ligand. Thus, it was demonstrated that the autoantibodies in UC patients specifically recognize integrin αvβ6.

### <Example 9. Antibody removal performance test of integrin αvβ6 immobilized by NHS covalent bonding>

Adsorption tests of the monoclonal autoantibody derived from an ulcerative colitis patient and the anti-integrin αvβ6 antibodies contained in the serum of an ulcerative colitis patient were conducted using an adsorbent in which integrin αvβ6 protein was immobilized to magnetic agarose using N-hydroxysuccinimide (NHS) covalent bonding.

Integrin αvβ6 (ACRO Biosystems, IT6-H52E1) in an amount of 3 to 30 µg was mixed with 25 µL (containing 5 µL of carrier) of NHS mag Sepharose (Cytiva, 28-9440-09) and 500 ng of the monoclonal autoantibody derived from an ulcerative colitis patient (UC antibody 1 prepared in Example 2) or 25 µL of the serum derived from an ulcerative colitis patient (UC patient 11 in Example 7), and a total of 500 µL of solution was reacted by inversion mixing at 4°C for 12 hours. After the reaction, a tube was placed on a magnetic rack to recover the reaction solution. As a control, a mixture to which no integrin αvβ6 was added was used to perform the similar reaction.

In the same manner as described in Example 7, the concentration of anti-integrin αvβ6 antibody contained in the recovered solution was determined, and the adsorption rate of anti-integrin αvβ6 antibody in the antibody adsorption reaction was calculated. Results are shown in Figure 14.

As is clear from Figure 14, the adsorbed amounts of UC antibody 1 and the anti-integrin αvβ6 antibody contained in the serum derived from UC patient 11 increased with an increase in the amount of integrin αvβ6 added for antibody adsorption.

### <Example 10. Antibody removal performance test of integrin αvβ6 immobilized on various carriers by NHS covalent bonding>

Integrin αvβ6 was immobilized on a cellulose carrier or a polymethyl methacrylate (PMMA) carrier by NHS covalent bonding, and antibody adsorption tests were performed. The cellulose and PMMA carriers were prepared as follows.

### Preparation of cellulose carrier:

Celphere C P-305 (Asahi Kasei) was used as the cellulose carrier. In a flask reaction vessel, 1 g of the cellulose carrier was placed, and suspended in a 0.05 M sodium phosphate buffer (90 mL, pH 6.8). Then, TEMPO (2,2,6,6-tetramethylpiperidine 1-oxyl, 0.016 g, 0.1 mmol) and sodium chlorite (80%, 1.13 g, 10 mmol) were added to a suspension, followed by gentle stirring. Next, a mixture of a 2M sodium hypochlorite aqueous solution (0.5 mL, 1.0 mmol) and a 0.05M sodium phosphate buffer (0.5 mL, pH 6.8) was added to the reaction suspension and mixed, followed by heating reaction at 60°C for 2, 6, 24, 48, and 72 hours. After each reaction time elapsed, the suspension was returned to room temperature and then filtered. A filtered substance was washed three times with distilled water and subjected to aspiration for 30 minutes to obtain crystalline cellulose beads with COOH groups attached to the surfaces. One gram of the carrier was filtered and washed twice with 10 mL of a 0.05 M 2- (N-morpholino)ethanesulfonic acid monohydrate (MES) buffer solution at pH 5.0 to prepare 20 mL of a 50 mg/mL suspension of the carrier in the MES buffer solution. In the MES buffer, 1-(3-dimethylaminopropyl)-3-ethylcarbodiimide hydrochloride (EDC) was dissolved to prepare 640 µL of a 150 mM solution. Sulfo NHS (sodium N-hydroxysulfosuccinimide) was dissolved in the MES buffer to prepare 320 µL of a 300 mM solution. The EDC solution and the sulfo-NHS solution were added in this order to the carrier suspension, and the mixture was shaken and stirred at room temperature for 1 hour. The mixture was then filtered and washed with a 0.1 M phosphate buffer (pH 7.4) to obtain an NHS-activated carrier.

### Preparation of PMMA carrier:

Methyl methacrylate (MMA) and methacrylic acid (MAA) were copolymerized by a dispersion polymerization method to obtain polymethyl methacrylate (PMMA) particles with introduced COOH groups. Fifty grams of MMA (M0087; Tokyo Chemical Industry Co., Ltd.) and 50 g of MAA (M0079; Tokyo Chemical Industry Co., Ltd.) were mixed with 116 g of purified water (161-08247; Fuji Film Wako Co., Ltd.) and 464 g of methanol (131-01826; Fuji Film Wako Co., Ltd.). Further, 10 g of polyvinylpyrrolidone (P0691; Tokyo Chemical Industry Co., Ltd.) as a dispersion stabilizer and 2 g of 2,2-azodiisobutyronitrile (A0566; Tokyo Chemical Industry Co., Ltd.) as a polymerization initiator were mixed in the polymerization flask, and reacted at 60°C for 6 hours while being stirred at 120 rpm. After the reaction, the particles were washed and the resulting particles were subjected to testing. Thereafter, an NHS-activated carrier was obtained in the same manner as described for the cellulose carrier.

### Immobilization of integrin αvβ6 protein onto NHS-activated carrier:

The required amount of carrier was weighed out and washed with 1 mM hydrochloric acid. The carrier and an integrin αvβ6 solution (500 µg/mL; ACRO Biosystems, IT6-H52E1) were mixed in a volume ratio of 2:1, and reacted by inversion mixing at room temperature for 2 hours. After the reaction, the solution was replaced with a 0.5 M ethanolamine solution (pH 8.3, containing 500 mM NaCl) for blocking, followed by inversion mixing at room temperature for 2 hours. Then, to remove excess reagents and reaction by-products, the mixture was alternately washed with 0.1 M Tris-HCl buffer (pH 8.3, containing 500 mM NaCl) and 0.1 M acetate buffer (pH 4.0, containing 500 mM NaCl), and finally replaced with a 50 mM Tris-HCl, 150 mM NaCl (pH 7.5) solution and stored at 4°C as a 50% slurry.

The two types of adsorbents thus obtained were used to perform an adsorption test of the monoclonal autoantibody derived from an ulcerative colitis patient (UC antibody 1 prepared in Example 2) in the same manner as described in Example 9, and the adsorption rate was calculated. Results are shown in Table 6. For comparison, Table 6 also shows the adsorption rate of an agarose carrier (NHS mag Sepharose; Cytiva, 28-9440-09) on which the same amount of integrin αvβ6 (10 µg per 5 µL of carrier) shown in Example 9 was immobilized.

**[Table 6]**

| Carrier material | Adsorption rate |
|---|---|
| agarose | 48% |
| cellulose | 45% |
| PMMA | 43% |

### <Example 11. Antibody removal test of integrin αvβ6 immobilized by covalent bonding other than NHS method>

An adsorption test of the monoclonal autoantibody derived from an ulcerative colitis patient (UC antibody 1 prepared in Example 2) was conducted using an adsorbent in which integrin αvβ6 was immobilized on a carrier activated by a formyl method, an epoxy method, or an EAH method. Each carrier was prepared as follows.

### Method for preparing formyl group-immobilized carrier:

An integrin αvβ6 solution (500 µg/mL; ACRO Biosystems, IT6-H52E1) was mixed with formyl group-activated cellulose (19853: JNC) in a volume ratio of 1:1, and reacted by inversion mixing at room temperature for 1 hour. To each tube was added 2 mg of trimethylamine borane (CH₃) ₃NBH₃, followed by inversion mixing at room temperature for 2 hours. Through the reaction, an exposed amino group of integrin αvβ6 protein forms a covalent bond with the formyl group of the cellulose carrier, thereby immobilizing the protein on the surface of the carrier. Furthermore, in order to block unreacted formyl groups on the carrier surface, the immobilized carrier was immersed in a 200 mM Tris-HCl buffer (pH 7.0) containing 1 M ethanolamine, and reacted by inversion mixing at room temperature for 2 hours. After the reaction, the solvent of the carrier was replaced with a solution of 50 mM Tris-HCl and 150 mM NaCl (pH 7.5), and the carrier was stored at 4°C as a 50% slurry.

### Method for preparing epoxy group-immobilized carrier:

Epoxy-activated Sepharose 6B (Cytiva, 17048001) was used as an epoxy group-activated carrier. The required amount of carrier was weighed out, swollen with distilled water, and washed with a neutral buffer on a glass filter. The carrier and an integrin αvβ6 solution (500 µg/mL; ACRO Biosystems, IT6-H52E1) were mixed in a volume ratio of 1:1 and subjected to shaking reaction at room temperature for 16 hours. After the reaction, the solution was replaced with a 1 M ethanolamine solution at pH 8.0 for blocking, followed by still standing at 40°C for 4 hours. Then, to remove excess reagents and reaction by-products, the mixture was alternately washed with a 0.1 M Tris-HCl buffer (pH 8.0, containing 500 mM NaCl) and a 0.1 M acetate buffer (pH 4.0, containing 500 mM NaCl), and finally replaced with a 50 mM Tris-HCl, 150 mM NaCl (pH 7.5) solution and stored at 4°C as a 50% slurry.

### Method for preparing EAH group-immobilized carrier:

EAH Sepharose (registered trademark) 4B (Cytiva, 17056901) was used as an EAH group carrier. The required amount of carrier was washed on a glass filter with distilled water adjusted to pH 4.5 with hydrochloric acid, and then washed with 500 mM NaCl. The carrier and an integrin αvβ6 solution (500 µg/mL; ACRO Biosystems, IT6-H52E1) were mixed in a volume ratio of 2:1 and reacted overnight at 4°C. After the reaction, in order to remove excess reagents and reaction by-products, the mixture was alternately washed with a 0.1 M Tris-HCl buffer (pH 8.0, containing 500 mM NaCl) and a 0.1 M acetate buffer (pH 4.0, containing 500 mM NaCl), and finally replaced with a 50 mM Tris-HCl, 150 mM NaCl (pH 7.5) solution and stored at 4°C as a 50% slurry.

The carrier on which integrin αvβ6 was immobilized or an albumin immobilized carrier (control) (13 µg) was mixed with 500 ng of the monoclonal autoantibody derived from an ulcerative colitis patient (UC antibody 1), and a total of 500 µL of mixture solution was reacted by inversion mixing at 4°C for 12 hours. Then, the reaction solution was recovered.

The concentration of anti-integrin αvβ6 antibody contained in the recovered solution was determined in the same manner as described in Example 7 except that an HRP-labeled anti-human IgG antibody (ab98535, 40,000-fold diluted) was used as the labeled antibody, and the adsorption rate of anti-integrin αvβ6 antibody in the antibody adsorption reaction was calculated. Results are shown in Table 7.

**[Table 7]**

| Carrier material | Carrier-activation method | Adsorption rate |
|---|---|---|
| cellulose | formyl method | 33% |
| agarose | epoxy method | 56% |
| agarose | EAH method | 54% |

### <Example 12. Antibody removal test of integrin αvβ6 immobilized by avidin-biotin binding>

Integrins with biotin attached to the C-terminus of either subunit (α chain or β chain) can be bound to an avidin-immobilized carrier with a binding affinity equivalent to that of a covalent bond. In this binding mode, the integrin is immobilized upright on the carrier, thereby exposing the head portion which is believed to be a recognition site for many autoantibodies, and thus it is expected to increase the efficiency of interaction with autoantibodies. An adsorption test of the monoclonal autoantibody derived from an ulcerative colitis patient (UC antibody 1 prepared in Example 2) was carried out using an avidin-attached carrier on which biotinylated integrin αvβ6 was immobilized.

### (1) Antibody removal test of NeutrAvidin agarose-immobilized integrin αvβ6

After 400 µL (200 µL as carrier) of NeutrAvidin agarose (Thermo Fisher Scientific, 29200) was washed with a binding buffer (25 mM Tris, 0.15 M sodium chloride, pH 7.2), 200 µL (80 µg) of biotinylated integrin αvβ6 (ACRO Biosystems, IT6-H82E4) was mixed and left to stand at room temperature for 15 minutes. A supernatant was then removed. The immobilized gel was washed with a buffer (20 mM Tris, 150 mM NaCl, pH 7.4) and stored at 4°C as a 50% slurry. As a control, biotinylated bovine serum albumin (SIGMA, A8549) was immobilized on NeutrAvidin in the same way to prepare an agarose carrier (BSA). Biotinylated human integrin αvβ6 (ACROBiosystems, product number IT6-H82E4) is a heterodimeric protein comprising an α chain and a β chain, wherein the α chain comprises an extracellular region extending from Phe at position 31 to Val at position 992 in the amino acid sequence of the human integrin αv chain, and an acidic tail sequence, a polyhistidine tag, and an Avi tag (registered trademark) at its C-terminus, and the β chain comprises an extracellular region extending from Gly at position 22 to Asn at position 707 in the amino acid sequence of the human integrin β6 chain, and a basic tail sequence at its C-terminus.

A mixture of 500 ng of the anti-integrin αvβ6 antibody (UC antibody 1) with 10 µL or 30 µL of the above-mentioned carrier-immobilized integrin αvβ6 or BSA (immobilized amount: 2 µg or 7 µg, respectively) was reacted in a total volume of 500 µL by inversion mixing at 4°C for 12 hours. After the reaction, the reaction solution was left to stand and then recovered.

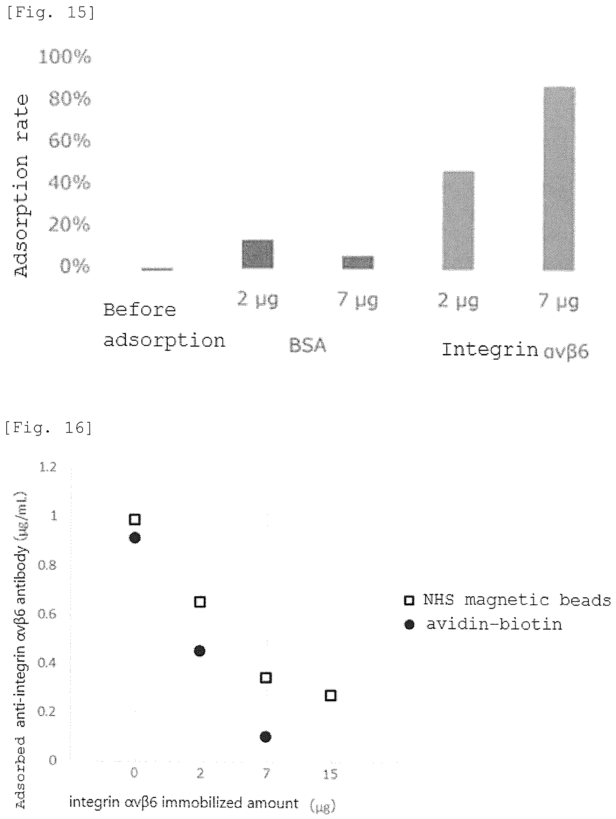

The concentration of anti-integrin αvβ6 antibody contained in the recovered solution was determined in the same manner as described in Example 7 except that an HRP-labeled anti-human IgG antibody (40,000-fold diluted; ab98535; Abcam) was used as a labeled antibody, and the adsorption rate of anti-integrin αvβ6 antibody in the antibody adsorption reaction was calculated according to a formula as below. In this Example, the antibody concentration before the adsorption reaction (Pre-antibody concentration) is 1 µg/mL (=500 ng/500 µL). Results are shown in Figure 15. Adsorption rate (%) = [{antibody concentration before adsorption reaction (Pre-antibody concentration) - antibody concentration after adsorption reaction} / antibody concentration before adsorption reaction (Pre-antibody concentration)] x 100

As a result, the adsorbents on which 2 µg and 7 µg of integrin αvβ6 were immobilized had antibody adsorption rates of 48% and 88%, respectively (Figure 15). The carrier on which BSA was immobilized (control) had an adsorption rate of 14% at maximum. Thus it was found that the anti-integrin αvβ6 antibody was specifically adsorbed onto the integrin αvβ6 immobilized carriers.

### (2) Antibody removal test of integrin αvβ6 immobilized using various carriers or various avidins

As various carriers other than agarose, a cellulose carrier and a PMMA carrier were prepared in the same manner as described in Example 10, and 1 mg of NeutrAvidin (Thermo Fisher) was immobilized on 100 µL of carrier. Furthermore, as immobilization carriers using various avidins other than NeutrAvidin, StreptAvidin agarose (Thermo Fisher) which is StreptAvidin-immobilized agarose, and Avidin agarose (Thermo Fisher) which is Avidin-immobilized agarose were used.

These carriers thus obtained were used to immobilize biotinylated integrin αvβ6 (ACRO Biosystems, IT6-H82E4), and antibody adsorption tests were carried out, in the same manner as described in Example 12(1). Results are shown in Table 8. For comparison, Table 6 also shows the adsorption rate of NeutrAvidin agarose-immobilized integrin αvβ6 shown in Examle 12(1).

**[Table 8}**

| Carrier material | Substance attached to the carrier | Adsorption rate |
|---|---|---|
| agarose | NeutrAvidin | 88% |
| cellulose | NeutrAvidin | 80% |
| PMMA | NeutrAvidin | 82% |
| agarose | StreptAvidin | 84% |
| agarose | Avidin | 82% |

### (3) Comparison of avidin-biotin method and covalent bonding method

Monoclonal antibody adsorption tests were carried out under the conditions as described in Example 12(1) using the adsorbent in which biotinylated integrin αvβ6 was immobilized on NeutrAvidin-immobilized agarose as prepared in Example 12(1), and the adsorbent in which integrin αvβ6 was immobilized on NHS mag Sepharose (Cytiva) as prepared in Example 9, and their performance was compared. Results are shown in Figure 16.

As is clear from Figure 16, the adsorbent in which biotinylated integrin αvβ6 was immobilized on NeutrAvidin-immobilized agarose had superior antibody adsorption performance to the adsorbent in which the integrin was immobilized by the covalent bonding method.

### <Example 13. Antibody removal test using circulation column>

A circulation adsorption test of a monoclonal autoantibody solution derived from an ulcerative colitis patient was carried out using a column packed with the NeutrAvidin-biotinylated integrin αvβ6-immobilized agarose adsorbent as prepared in Example 12.

### Circulation column:

Figure 16 shows an overview of an example of a circulation column. Ten milliliters (antibody concentration: 1 µg/mL) of a solution containing 10 µg of the monoclonal autoantibody derived from an ulcerative colitis patient (UC antibody 1 prepared in Example 2) and 0.1 g of human serum albumin (SIGMA, A8549) was prepared. The antibody solution was circulated by a peristaltic pump, and passed through a column containing an agarose adsorbent onto which biotinylated integrin αvβ6 (10 µg) was immobilized, which had been prepared in the same manner as in Example 12. The antibody solution was collected every 30 minutes during circulation. As a control, a similar test was carried out using an adsorbent on which 10 µg of biotinylated bovine serum albumin (SIGMA, A8549) was immobilized.

### Measurement of anti-integrin αvβ6 antibody concentration:

The concentrations of anti-integrin αvβ6 antibody contained in the solutions sampled during the circulation test and in the solutions after the inversion mixing reaction were measured by ELISA. A microtiter plate was coated with 100 µL/well of 2 µg/mL integrin αvβ6 overnight at 4°C, followed by washing and blocking. After washing, the column-passed sample was diluted 10-fold and then added to the plate at 100 µL/well, followed by incubation at room temperature for 60 minutes. After washing, an HRP-labeled anti-human IgG antibody (40,000-fold dilution; ab98535; Abcam) was added to the plate at 100 µL/well, and the plate was incubated at room temperature for 60 minutes. After washing, the plate was incubated with 3,3',5,5'-tetramethylbenzidine for 7 minutes, and the absorbance density (OD value) at 450 nm was measured to detect human IgG bound to integrin αvβ6 on the solid phase.

### Conversion of anti-integrin αvβ6 antibody concentration:

To a microtiter plate comprising 2 µg/mL of solid-phased integrin αvβ6, 1 to 0.001 µg/mL of UC antibody 1 (see Table 1) prepared in Example 2 and diluted in a 3-fold common ratio was added, and incubated at room temperature for 60 minutes. After washing the plate, 100 µL/well of an HRP-labeled anti-human IgG antibody (40,000-fold dilution; ab98535; Abcam) as a secondary antibody was added to the plate, and the plate was incubated at room temperature for 60 minutes. Then, the plate was incubated with 3,3',5,5'-tetramethylbenzidine for 7 minutes, and the optical density at 450 nm (OD value) was measured. Next, a calibration curve was prepared to determine the concentration of anti-integrin αvβ6 antibodies from the obtained OD values. Using the calibration curve, the concentration of anti-integrin αvβ6 antibodies contained in the solution that had been subjected to the antibody adsorption reaction was determined. The adsorption rate of autoantibodies was calculated by dividing a reduction in the amount of autoantibodies after passing through each column by the amount of autoantibodies after passing through a dummy column filled with no adsorbent (referred to as "pass-through"), based on the following formula. Adsorption rate (%) = [{ (pass-through antibody concentration) - (antibody concentration of adsorbed sample)}/(pass-through antibody concentration)] × 100

### Results:

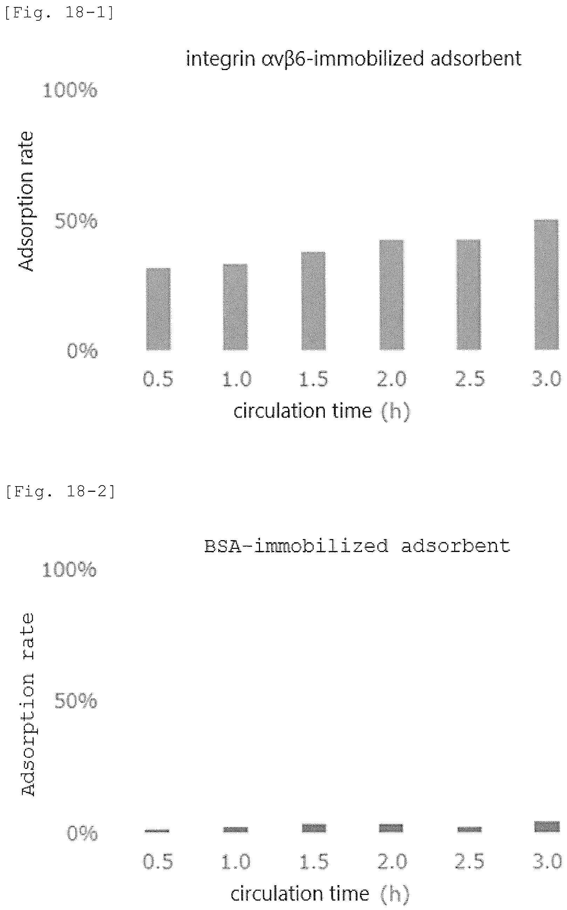

Results are shown in Figures 18-1 and 18-2. As shown in Figures 18-1 and 18-2, when the antibody solution sample was circulated through the integrin αvβ6-immobilized adsorbent, the antibody was adsorbed over time, and the adsorption rate reached 50% after 3 hours. Approximately 5 µg of the anti-integrin αvβ6 antibody in the antibody solution was adsorbed to 10 µg of integrin αvβ6 immobilized on the carrier. In the control (BSA), almost no adsorption of anti-integrin αvβ6 antibody was observed during circulation for 3 hours. Therefore, it was found that the anti-integrin αvβ6 antibody was specifically removed by the integrin αvβ6-immobilized adsorbent in the column.

### <Example 14. Measurement of amount of anti-integrin αvβ6 antibody in patient serum>

Based on the anti-integrin αvβ6 antibody concentration measurement and conversion method as described in Example 7, the concentrations of anti-integrin αvβ6 antibodies in the serum of UC (n=8) and PSC (n=13) patients were quantified. As a result, the mean levels of anti-integrin αvβ6 antibodies in the sera of UC and PSC patients were approximately 5.8 µg/mL and 8.0 µg/mL, respectively (Table 9). Of 21 patients' sera in total measured, 17 patients had a level below 10 µg/mL.

**[Table 9]**

| | Anti-integrin αvβ6 antibody level (µg/mL) |
|---|---|
| UC patient | 5.8 ± 3.4 |
| PSC patient | 8.0 ± 15.0 |

### <Example 15. Verification of direct pathological effects of anti-integrin αvβ6 antibody - 1>

This Example was to check whether passive transfer of anti-integrin αvβ6 antibody into animals by using a serum from a human UC patient or a PSC patient can reproduce the characteristics of the diseases. The amounts of anti-integrin αvβ6 antibodies contained in the UC patient-derived serum, PSC patient-derived serum, and control (healthy individual) serum used in this Example are as shown in the following table.

**[Table 10]**

| | Anti-integrin αvβ6 IgG level (A450) |
|---|---|
| US patient serum | 3.028 |
| PSC patient serum | 3.003 |
| control serum | 0.153 |

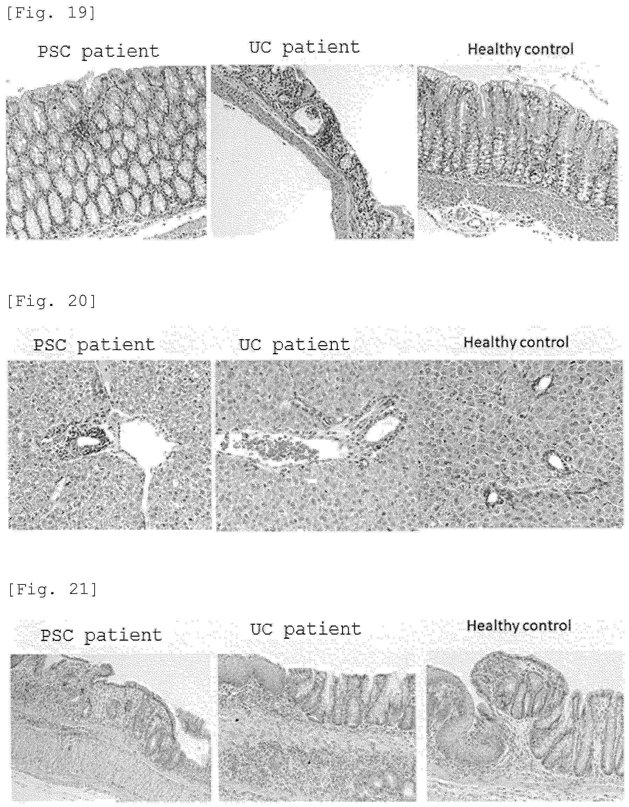

One-week-old mice (Balb/c, C57BL/6) were subcutaneously injected in the back with 2 ml/mouse of the UC patient serum, PSC patient serum, or healthy control serum as a control. After 24 hours, the mice were sacrificed, and the large intestines and bile ducts of the mice were observed by hematoxylin-eosin (HE) staining. As a result, the administration of UC patient serum caused crypt distortion and cellular infiltration in the large intestine (Figure 19). Furthermore, the administration of PSC patient serum resulted in cellular infiltration in some areas around the bile duct (Figure 20).

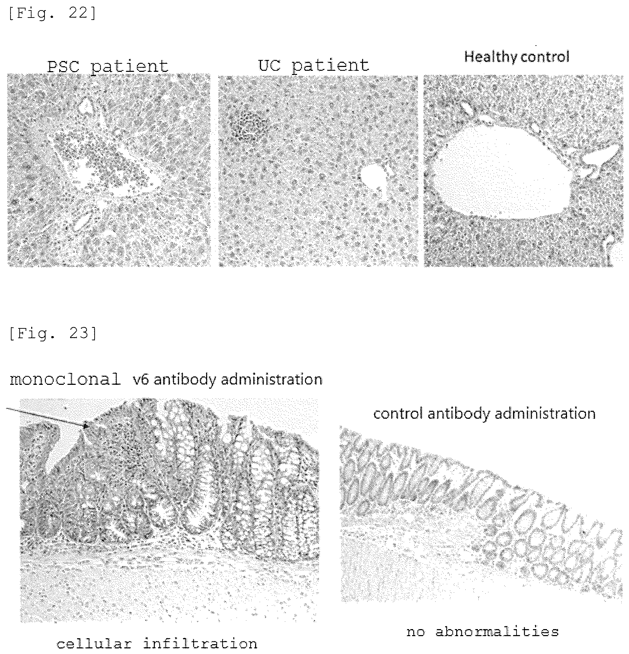

After 1% DSS was administered to 8-week-old mice (Balb/c, C57BL/6) for 3 weeks, the mice received no drug for 3-weeks. The above-mentioned amount of DSS causes mild inflammation in the intestines, and it is believed that the inflammation is completely cured by discontinuing the drug for about three weeks. Next, 1 ml/mouse of the UC patient serum, PSC patient serum, or healthy control serum as a control was administered by subcutaneous injection into the back of the mice three times every 24 hours. The mice were sacrificed 24 hours after the final administration, and the large intestines and bile ducts of the mice were observed by HE staining. As a result, in both the UC patient serum administration group and the PSC patient serum administration group, significant cellular infiltration was observed under the colonic mucosa, and crypt distortion was also observed, as compared to the control group (Figure 21). Furthermore, the administration of PSC patient serum induced significant fibrosis around the bile duct (Figure 22).

Therefore, it was demonstrated that the presence of anti-integrin αvβ6 antibodies in the sera of UC and PSC patients leads to the onset of UC and PSC pathology. Furthermore, it was found that animal models exhibiting the symptoms of UC or PSC can be produced by administration of the serum from a UC patient or a PSC patient.

### <Example 16. Verification of direct pathological effects of anti-integrin αvβ6 antibody - 2>

This Example was to check whether passive transfer of anti-integrin αvβ6 monoclonal antibody from UC human patients into animals can reproduce the characteristics of UC.

Mice (B6 wild type) received UC antibody 3 or UC antibody 4 as prepared in Example 2 (see Table 1) or healthy human IgG (Fujifilm Wako Pure Chemical Industries, 149-09503) as a control at 10 mg/mouse by subcutaneous injection into the back twice a week for three weeks. The mice were sacrificed 24 hours after the final administration, and the large intestines of the mice were observed by HE staining. As a result, cellular infiltration was observed in the large intestines of the mice administered with the anti-integrin αvβ6 monoclonal antibody. The result of administration of UC antibody 4 is shown in Figure 23. On the other hand, no abnormalities were observed in the large intestines of the mice administered with the control antibody (Figure 23).

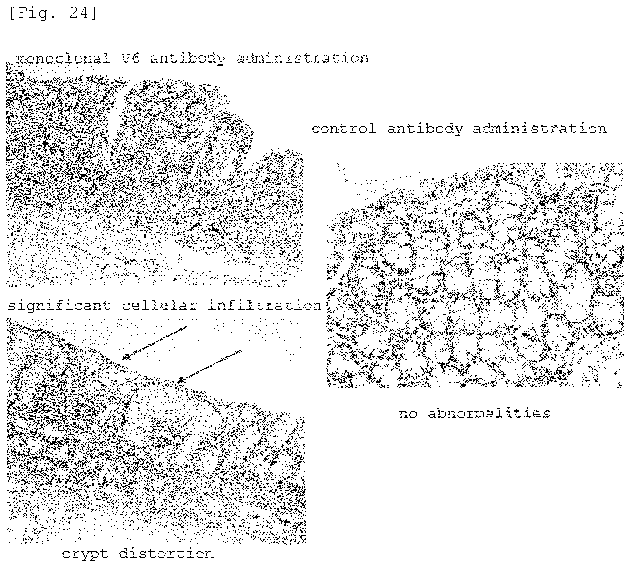

After 1% DSS was administered to mice (B6 wild type) for 3 weeks, no drug was administered to the mice for 6 weeks. Next, both UC antibody 3 and UC antibody 4 as prepared in Example 2, or UC antibody 3 or UC antibody 4 (see Table 1), or healthy human IgG (Fujifilm Wako Pure Chemical Industries, 149-09503) as a control were administered at 0.1 mg/mouse by subcutaneous injection into the back twice a week for three weeks. The mice were sacrificed 24 hours after the final administration, and the large intestines of the mice were observed by HE staining. As a result, significant cellular infiltration and crypt distortion were observed in the large intestines of the mice administered with anti-integrin αvβ6 monoclonal antibodies (both or either of UC antibody 3 and UC antibody 4). The results of administration of both UC antibody 3 and UC antibody 4 are shown in Figure 24. On the other hand, no abnormalities were observed in the large intestines of the mice administered with the control antibody (Figure 24).

Thus, it was shown that administration of anti-integrin αvβ6 monoclonal antibody derived from a UC patient to mice resulted in pathology similar to UC. Thus, the pathogenicity of anti-integrin αvβ6 monoclonal antibodies derived from UC patients was demonstrated, and it was shown that the presence of the antibodies leads to the onset of UC pathology. Furthermore, it was found that an animal model exhibiting the symptoms of UC can be produced by administering an anti-integrin αvβ6 monoclonal antibody derived from a UC patient. Furthermore, based on the results of this Example and Example 14, it is clear that anti-integrin αvβ6 antibodies derived from PSC patients similarly cause the onset of PSC pathology, and that administration of the antibodies makes it possible to produce PSC animal models.

## Claims

1. A system for treating ulcerative colitis or primary sclerosing cholangitis, the system comprising a means for removing an anti-integrin αvβ6 antibody or an anti-integrin αvβ6 antibody-producing B cell that produces the anti-integrin avpf6 antibody, wherein the anti-integrin αvβ6 antibody has activity competing with fibronectin for binding to integrin αvβ6 and is specifically produced in a subject suffering from ulcerative colitis or primary sclerosing cholangitis.

2. The system according to claim 1, wherein the means comprises a substance that specifically binds to the anti-integrin αvβ6 antibody or the anti-integrin αvβ6 antibody-producing B cell.

3. The system according to claim 2, wherein the substance is a fragment or an entirety of integrin αvβ6 protein.

4. The system of claim 3, which comprises a column carrying a fragment or an entirety of integrin αvβ6 protein.

5. A solid carrier for the treatment of ulcerative colitis or primary sclerosing cholangitis, the solid carrier comprising a fragment or an entirety of integrin αvβ6 protein capable of specifically binding to an anti-integrin αvβ6 antibody or an anti-integrin αvβ6 antibody-producing B cell that produces the anti-integrin αvβ6 antibody, and thereby being capable of adsorbing the anti-integrin αvβ6 antibody or the anti-integrin αvβ6 antibody-producing B cell, wherein the anti-integrin αvβ6 antibody has activity competing with fibronectin for binding to integrin αvβ6 and is specifically produced in a subject suffering from ulcerative colitis or primary sclerosing cholangitis.

6. The solid carrier according to claim 5, wherein the anti-integrin αvβ6 antibody comprises a heavy chain CDR2 or CDR3 comprising any one amino acid sequence selected from RGD, RGRD, or RGSGD, and the fragment or entirety of integrin αvβ6 protein has a binding dissociation constant (KD value) of 100 nM or less for the anti-integrin αvβ6 antibody.

7. The solid carrier according to claim 5 or 6, wherein the integrin αvβ6 protein is immobilized at a weight equal to or greater than twice the weight of the antibody to be captured, or the fragment of integrin αvβ6 protein is immobilized at a weight equal to or greater than a value obtained by multiplying twice the weight of the antibody to be captured by a proportion of the molecular weight of the fragment to the molecular weight of the entirety of integrin αvβ6.

8. A column comprising the solid carrier according to any one of claims 5 to 7.

9. The column according to claim 8, which comprises the solid carrier such that the weight of the integrin αvβ6 protein per column is at least twice the weight of the antibody to be captured, or the weight of the fragment of integrin αvβ6 protein per column is at least a value obtained by multiplying twice the weight of the antibody to be captured by a proportion of the molecular weight of the fragment to the molecular weight of the entirety of integrin αvβ6.

10. The column according to claim 8 or 9, which is used for apheresis.

11. A method for producing an animal model of primary sclerosing cholangitis, the method comprising immunizing a non-human animal with a fragment or an entirety of integrin αvβ6 protein.

12. A method for producing an animal model of primary sclerosing cholangitis, the method comprising knocking out an integrin β6 gene in a non-human animal.

13. A method for producing an animal model of an ulcerative colitis or primary sclerosing cholangitis, the method comprising administering to a non-human animal an anti-integrin αvβ6 antibody derived from a patient with ulcerative colitis or primary sclerosing cholangitis.

14. The method according to claim 13, wherein the antibody is a serum or a monoclonal antibody.

15. An animal model of ulcerative colitis having a symptom of ulcerative colitis.

16. An animal model of primary sclerosing cholangitis having a symptom of primary sclerosing cholangitis.

17. The animal model of primary sclerosing cholangitis according to claim 16, which is a knockout mouse lacking the function of integrin αvβ6.
